Europäisches Patentamt

European Patent Office

Office européen des brevets

(19)

(11) **EP 1 384 782 A1**

(12) **EUROPEAN PATENT APPLICATION**
published in accordance with Art. 158(3) EPC

(43) Date of publication:
    **28.01.2004 Bulletin 2004/05**

(21) Application number: **02705018.6**

(22) Date of filing: **22.03.2002**

(51) Int Cl.⁷: **C12N 15/31**, C12Q 1/02,
    C12Q 1/68

(86) International application number:
    **PCT/IB2002/000890**

(87) International publication number:
    **WO 2002/079476 (10.10.2002 Gazette 2002/41)**

(84) Designated Contracting States:
    **AT BE CH CY DE DK ES FI FR GB GR IE IT LI LU
    MC NL PT SE TR**
    Designated Extension States:
    **AL LT LV MK RO SI**

(30) Priority: **30.03.2001 JP 2001098371**

(71) Applicants:
    • **National Institute of Advanced Industrial
      Science and Technology
      Tokyo 100-8921 (JP)**
    • **National Research Institute of Brewing
      Higashihiroshima-shi, Hiroshima 739-0046 (JP)**
    • **National Food Research Institute
      Tsukuba-shi, Ibaraki 305-8642 (JP)**

(72) Inventors:
    • **MACHIDA, Masayuki
      Tsukuba-shi, Ibaraki 305-0051 (JP)**
    • **AKITA, Osamu
      Higashihiroshima-shi, Hiroshima 739-0025 (JP)**

    • **KASHIWAGI, Yutaka
      Tsukuba-shi, Ibaraki 305-0031 (JP)**
    • **KITAMOTO, Katsuhiko
      Bunkyo-ku, Tokyo 113-0032 (JP)**
    • **HORIUCHI, Hiroyuki
      Shinjuku-ku, Tokyo 160-0022 (JP)**
    • **TAKEUCHI, Michio
      Hachioji-shi, Tokyo 193-0934 (JP)**
    • **KOBAYASHI, Tetsuo, Takinomizu-jutaku 7-201
      Nagoya-shi, Aichi 458-0015 (JP)**
    • **KITAMOTO, Noriyuki
      Nagoya-shi, Aichi 463-0003 (JP)**
    • **GOMI, Katsuya
      Sendai-shi, Miyagi 989-3201 (JP)**
    • **ABE, Keietsu
      Shiogama-shi, Miyagi 985-0043 (JP)**

(74) Representative: **Maschio, Antonio, Dr.
    D Young & Co,
    21 New Fetter Lane
    London EC4A 1DA (GB)**

(54) **METHOD OF DETECTING THE EXPRESSION OF ASPERGILLUS GENE**

(57)    The present invention provides a method of detecting the expression of a koji mold gene, and a DNA for specifically assaying the fermentation conditions for a koji mold. Specifically, the DNA for specifically assaying the fermentation conditions for a koji mold of the present invention may be characterized in that the DNA can be expressed in a koji mold when the koji mold is cultured under at least one culture condition selected from the group consisting of nutrient-rich culture, nutrient-deficient culture, solid culture, early germination culture, alkaline culture, high temperature culture and low temperature culture conditions.

**EP 1 384 782 A1**

**Description**

TECHNICAL FIELD

The present invention relates to a method of detecting the expression of a filamentous fungus gene and a DNA for specifically assaying fermentation conditions for filamentous fungi.

BACKGROUND ART

In fermentation production using a koji mold, it is important to monitor in detail the growth states and the expression of various fermentation functions (or genes) and the like of a koji mold. Furthermore, the processes of fermentation production do not always rely exclusively on the function of a sole strain of microorganisms, but may employ a plurality of different strains of microorganisms that are evolutionarily related each other. In addition, the fermentation production processes are passively contaminated with these microorganisms from the natural environment without artificially inoculating these microorganisms. These microorganisms to be contaminated are essential in many cases for fermentation production, and their contamination is originally expected. Thus, there are many cases where contamination itself cannot be prevented. In the meantime, in the natural environment, a number of microorganisms unfavorable for fermentation production are present, of which are biological species that are a very closely related evolutionarily to a koji mold. Accordingly in fermentation production, it is necessary to precisely assay the above items only for a koji mold from among many other microbial species that are difficult to be discriminated from a koji mold.

In the past, there was no method for directly monitoring these items. Observation by engineers (technical experts) using visual inspection or olfactory inspection via odor, or the like, or observation based on componential analysis or the like of organic acids, esters and the like in products have been performed, and then growth states and the expression of the fermentation functions have been inferred based on past experiences. However, these observations are nonobjective and indirect, so that the results of these observations are difficult to quantify. Thus, it has often been difficult to apply automation and laborsaving measures to be applied to these cases.

SUMMARY OF THE INVENTION

The purpose of the present invention is to provide a method of detecting the expression of a koji mold gene, and a DNA for specifically assaying the fermentation conditions for a koji mold.

As a result of intensive studies to solve the above problems, we have succeeded in detecting the growth state and the fermentation degree of a koji mold using many koji mold genes, and thus completed the present invention.

That is, the present invention relates to the following DNA (a) or (b):

(a) a DNA comprising a nucleotide sequence represented by any one of SEQ ID NOS: 1 to 6006; and
(b) a DNA hybridizing under stringent conditions to the DNA comprising the nucleotide sequence represented by any one of SEQ ID NOS: 1 to 6006, which can be expressed in filamentous fungi when the filamentous fungi are cultured under at least one culture condition selected from the group consisting of a nutrient-rich culture, nutrient-deficient culture, solid culture, early germination culture, alkaline culture, high temperature culture, low temperature culture and maltose culture conditions.

Furthermore, the present invention is a DNA that can be expressed in a koji mold when the koji mold is cultured under at least one culture condition selected from the group consisting of a nutrient-rich culture, nutrient-deficient culture, solid culture, early germination culture, alkaline culture, high temperature culture, low temperature culture and maltose culture conditions.

The above DNA can be expressed when a koji mold is cultured under at least one culture condition selected from the group consisting of the nutrient-rich culture, nutrient-deficient culture, solid culture, early germination culture, alkaline culture, high temperature culture, low temperature culture and maltose culture conditions.

Examples of such DNAs are as follows:

(a) a DNA that can be expressed in a koji mold when the koji mold is cultured under a nutrient-rich culture condition and comprises a nucleotide sequence represented by any one of the SEQ ID NOS. shown in the 1 st row of Table 1;
(b) a DNA that can be expressed in a koji mold when the koji mold is cultured under a nutrient-deficient culture condition and comprises a nucleotide sequence represented by any one of the SEQ ID NOS. shown in the 2nd row of Table 1;
(c) a DNA that can be expressed in a koji mold when the koji mold is cultured under a solid culture condition and comprises a nucleotide sequence represented by any one of the SEQ ID NOS. shown in the 3rd row of Table 1;

(d) a DNA that can be expressed in a koji mold when the koji mold is cultured under an early germination culture condition and comprises a nucleotide sequence represented by any one of the SEQ ID NOS. shown in the 4th row of Table 1;

(e) a DNA that can be expressed in a koji mold when the koji mold is cultured under an alkaline culture condition and comprises a nucleotide sequence represented by any one of the SEQ ID NOS. shown in the 5th row of Table 1;

(f) a DNA that can be expressed in a koji mold when the koji mold is cultured under a high temperature culture condition and comprises a nucleotide sequence represented by any one of SEQ ID NOS. shown in the 6th row of Table 1;

(g) a DNA that can be expressed in a koji mold when the koji mold is cultured under a low temperature culture condition and comprises a nucleotide sequence represented by any one of the SEQ ID NOS. shown in the 7th row of Table 1; and

(h) a DNA that can be expressed in a koji mold when the koji mold is cultured under a maltose culture condition and comprises a nucleotide sequence represented by any one of the SEQ ID NOS. shown in the 8th row of Table 1.

Furthermore, the present invention is a primer set of at least two primers for amplifying a koji mold gene, wherein the two primers are selected from the group consisting of nucleotide sequences prepared from the whole or partial regions of a plurality of DNAs selected from the above DNAs.

Furthermore, the present invention is a probe for detecting a filamentous fungus gene, which comprises a nucleotide sequence prepared from the whole or partial regions of a plurality of DNAs selected from the above DNAs.

Furthermore, the present invention is a method of detecting filamentous fungi, comprising synthesizing cDNA from RNA extracted from filamentous fungi, amplifying the cDNA using the above primer set, and detecting a filamentous fungus gene from the obtained amplification product.

Furthermore, the present invention is a method of detecting filamentous fungi, comprising hybridizing the above probe to RNA extracted from filamentous fungi, and detecting a filamentous fungus gene from the obtained product.

Furthermore, the present invention is a method of estimating the growth states or the fermentation function of filamentous fungi using the results obtained by the above detection method as an indicator.

Examples of filamentous fungi include microorganisms belonging to the genus *Aspergillus,* the genus *Penicillium,* the genus *Humicola,* the genus *Trichoderma,* the genus *Mucor* or the genus *Fusarium.* In addition, an example of a microorganism belonging to the genus *Aspergillus* is a koji mold (for example, *Aspergillus oryzae*).

The present invention will be described in detail as follows.

1. Introduction

The present invention is intended to solve the above problems using the nucleotide sequences of many koji mold genes. Specifically, by the use of a number of hybridization probes or PCR primers having the nucleotide sequences according to the present invention, the amount of a koji mold DNA and the expression amount of koji mold genes can be precisely measured. This makes it possible to directly and precisely measure the growth state or the expression of the fermentation function of a koji mold among many other microbial species present in the fermentation production processes. The present invention include almost all the nucleotide sequences of genes expressed under various representative fermentation production conditions, by which, for example, the expression of the fermentation function of a koji mold during the fermentation production processes can be monitored in detail. Even if microorganisms that are different from those being evolutionarily closely related to a koji mold is present in the fermentation production processes, more precise assay can be performed by analysis using the nucleotide sequences of a large number of genes than when assaying with a smaller number of genes. With this method, not only is it possible to realize automation and laborsaving that have been extremely difficult to achieve thus for, but also improvement of the reliability of fermentation production and standardization of fermentation production can be achieved. Thus, a large decrease in the production costs can be realized.

A koji mold is extensively used in the fermentation industry. In the present invention, culture conditions are determined in detail to improve the production efficiency. A koji mold-derived DNA is cloned, and then the nucleotide sequence thereof is used for designing and producing a probe for the hybridization or primers for PCR, in order to monitor in detail the fermentation state of a koji mold.

2. Cloning of a koji mold-derived DNA

A koji mold-derived mRNA can be prepared by techniques that are generally employed. For example, products resulting from fermentation by a koji mold is treated with guanidium thiocyanate, phenol reagent or the like to obtain total RNA. Then, poly (A+) RNA (mRNA) is obtained by the affinity column method or the batch method using poly U-sepharose or the like with oligo dT-cellulose or sepharose 2B as a carrier. After a single-stranded cDNA is synthesized using the thus obtained mRNA as a template, oligo dT primers and reverse transcriptase, a double-stranded cDNA is synthesized from the single-stranded cDNA. The thus obtained double-stranded cDNA is incorporated into appropriate cloning vectors to construct recombinant vectors. *Escherichia coli* or the like is transformed with the obtained recombinant vectors, and then transformants are selected for tetracycline resistance and ampicillin resistance as markers, so that cDNA libraries can be obtained.

Here, *Escherichia coli* can be transformed by, for example, a method wherein recombinant vectors are added to competent cells prepared by allowing co-existence with calcium chloride, magnesium chloride or rubidium chloride. When a plasmid is used as a vector, it is required to contain a drug resistance gene, such as a tetracycline resistance gene or an ampicillin resistance gene. In addition, cloning vectors other than plasmids, such as a λ phage, can also be used.

The nucleotide sequences of the obtained clones are determined. The nucleotide sequence can be determined by any known method, such as the Maxam-Gilbert's chemical modification method or the dideoxynucleotide-chain termination method using the M13 phage. In general, sequencing is performed using an autosequencer (for example, the Autosequencer (model ABI 377) manufactured by Applied Biosystem).

The all obtained nucleotide sequences are represented by SEQ ID NOS: 1 to 6006. In addition to those having the nucleotide sequences of SEQ ID NOS: 1 to 6006, the DNAs of the present invention also encompass DNAs that hybridize under stringent conditions to the DNAs having the nucleotide sequences of SEQ ID NOS: 1 to 6006, and are (a) DNAs which can be expressed in filamentous fungi when the filamentous fungi are cultured under at least one culture condition selected from the group consisting of a nutrient-rich culture, nutrient-deficient culture, solid culture, early germination culture, alkaline culture, high temperature culture, low temperature culture, and maltose culture conditions, or (b) DNAs which encode proteins having functions identical to those of the expression products of the DNAs. The term "stringent conditions" means conditions wherein specific hybrids are formed, but nonspecific hybrids are not formed. Under an example of such conditions, DNA having high homology (of 60% or more and preferably 80% or more) hybridizes. A more specific example of the conditions consists of a sodium concentration of 150 to 900 mM, preferably 600 to 900 mM, and temperature of 60 to 68°C.

Moreover, the DNAs of the present invention differ in their expression modes depending on the culture conditions. To show the relationship between culture conditions and DNAs to be expressed, and specifically, which DNA is expressed under which culture condition, culture conditions and SEQ ID NOS. are summarized in Table 1.

Table 1: DNA expressed under each culture condition    (SEQ ID NO:)

| 1st row | 2nd row | 3rd row | | | | | | 4th row | 5th row | 6th row | | 7th row | 8th row |
|---|---|---|---|---|---|---|---|---|---|---|---|---|---|
| nutrient-rich culture | nutrient-deficient culture | solid culture | | | | | | early germination culture | alkaline culture | high temperature culture | | low temperature culture | Maltose culture |
| SEQ ID NO: | SEQ ID NO: | SEQ ID NO: | | | | | | SEQ ID NO: | SEQ ID NO: | SEQ ID NO: | | SEQ ID NO: | SEQ ID NO: |
| 1 | 502 | 459 | 1270 | 459 | 2086 | 2604 | 3112 | 3626 | 4132 | 818 | 75 | 1791 | 5366 | 321 | 12 |
| 2 | 503 | 740 | 1271 | 741 | 2087 | 2605 | 3113 | 3627 | 4133 | 824 | 80 | 1820 | 5368 | 627 | 59 |
| 3 | 504 | 741 | 1272 | 742 | 2088 | 2606 | 3114 | 3628 | 4134 | 842 | 115 | 2055 | 5369 | 748 | 311 |
| 4 | 506 | 742 | 1273 | 745 | 2089 | 2607 | 3115 | 3629 | 4135 | 892 | 159 | 2292 | 5371 | 781 | 368 |
| 5 | 508 | 743 | 1274 | 773 | 2090 | 2609 | 3116 | 3630 | 4136 | 901 | 287 | 2317 | 5373 | 875 | 435 |
| 6 | 509 | 744 | 1275 | 781 | 2091 | 2610 | 3117 | 3631 | 4137 | 906 | 510 | 2514 | 5374 | 901 | 556 |
| 7 | 512 | 745 | 1277 | 782 | 2092 | 2611 | 3118 | 3632 | 4138 | 994 | 511 | 2536 | 5375 | 943 | 639 |
| 8 | 513 | 746 | 1278 | 788 | 2093 | 2612 | 3119 | 3633 | 4139 | 996 | 516 | 3112 | 5376 | 986 | 745 |
| 9 | 514 | 747 | 1279 | 796 | 2094 | 2613 | 3120 | 3634 | 4140 | 1004 | 523 | 4795 | 5378 | 1042 | 835 |
| 10 | 515 | 748 | 1280 | 800 | 2095 | 2615 | 3121 | 3635 | 4141 | 1025 | 669 | 4796 | 5379 | 1134 | 858 |
| 11 | 517 | 749 | 1281 | 802 | 2096 | 2616 | 3122 | 3636 | 4142 | 1032 | 670 | 4797 | 5380 | 1139 | 875 |
| 13 | 518 | 750 | 1282 | 806 | 2097 | 2617 | 3123 | 3637 | 4143 | 1042 | 671 | 4798 | 5381 | 1189 | 953 |
| 14 | 519 | 751 | 1283 | 818 | 2098 | 2618 | 3124 | 3638 | 4144 | 1048 | 672 | 4799 | 5382 | 1200 | 994 |
| 15 | 520 | 752 | 1284 | 840 | 2099 | 2619 | 3125 | 3639 | 4145 | 1066 | 673 | 4800 | 5383 | 1234 | 998 |
| 16 | 521 | 753 | 1285 | 841 | 2101 | 2620 | 3126 | 3640 | 4146 | 1078 | 674 | 4801 | 5384 | 1239 | 1012 |
| 17 | 522 | 754 | 1286 | 869 | 2103 | 2621 | 3127 | 3641 | 4147 | 1129 | 677 | 4802 | 5385 | 1261 | 1025 |
| 18 | 524 | 755 | 1287 | 874 | 2104 | 2623 | 3128 | 3642 | 4148 | 1139 | 678 | 4803 | 5387 | 1313 | 1065 |
| 19 | 525 | 756 | 1288 | 875 | 2105 | 2624 | 3129 | 3643 | 4149 | 1203 | 679 | 4804 | 5388 | 1373 | 1084 |
| 20 | 526 | 757 | 1289 | 877 | 2106 | 2625 | 3130 | 3644 | 4150 | 1304 | 683 | 4805 | 5389 | 1385 | 1119 |
| 21 | 527 | 758 | 1290 | 900 | 2108 | 2627 | 3131 | 3645 | 4151 | 1358 | 687 | 4806 | 5390 | 1389 | 1179 |
| 22 | 528 | 759 | 1292 | 901 | 2109 | 2628 | 3132 | 3646 | 4152 | 1483 | 688 | 4807 | 5391 | 1396 | 1275 |
| 23 | 529 | 760 | 1293 | 906 | 2110 | 2629 | 3133 | 3647 | 4153 | 1538 | 707 | 4808 | 5392 | 1398 | 1371 |
| 24 | 530 | 762 | 1294 | 907 | 2111 | 2630 | 3134 | 3648 | 4154 | 1559 | 718 | 4809 | 5393 | 1416 | 1377 |
| 25 | 531 | 763 | 1295 | 912 | 2112 | 2631 | 3135 | 3649 | 4155 | 1580 | 727 | 4810 | 5394 | 1423 | 1392 |
| 26 | 532 | 764 | 1296 | 921 | 2113 | 2633 | 3136 | 3651 | 4156 | 1645 | 828 | 4811 | 5397 | 1465 | 1393 |

EP 1 384 782 A1

| | | | | | | | | | | | | | | | |
|---|---|---|---|---|---|---|---|---|---|---|---|---|---|---|---|
| 27 | 1398 | 1466 | 5398 | 4812 | 875 | 1795 | 4157 | 3652 | 3137 | 2634 | 2114 | 928 | 1297 | 765 | 533 |
| 28 | 1401 | 1467 | 5399 | 4814 | 883 | 1796 | 4158 | 3653 | 3138 | 2635 | 2115 | 929 | 1298 | 766 | 534 |
| 29 | 1411 | 1468 | 5400 | 4815 | 919 | 1825 | 4159 | 3655 | 3139 | 2636 | 2116 | 931 | 1299 | 767 | 535 |
| 30 | 1423 | 1469 | 5401 | 4816 | 1056 | 1887 | 4160 | 3656 | 3140 | 2637 | 2117 | 935 | 1300 | 768 | 536 |
| 31 | 1449 | 1470 | 5402 | 4817 | 1093 | 1933 | 4161 | 3657 | 3141 | 2638 | 2118 | 947 | 1302 | 769 | 537 |
| 32 | 1466 | 1471 | 5403 | 4818 | 1096 | 1939 | 4162 | 3658 | 3142 | 2639 | 2119 | 951 | 1303 | 770 | 538 |
| 33 | 1500 | 1472 | 5404 | 4819 | 1121 | 1947 | 4163 | 3659 | 3143 | 2640 | 2121 | 967 | 1304 | 771 | 539 |
| 34 | 1506 | 1473 | 5405 | 4820 | 1223 | 2054 | 4164 | 3660 | 3144 | 2641 | 2122 | 980 | 1305 | 772 | 540 |
| 35 | 1515 | 1474 | 5406 | 4821 | 1240 | 2065 | 4165 | 3661 | 3145 | 2642 | 2123 | 981 | 1306 | 773 | 541 |
| 36 | 1550 | 1475 | 5407 | 4822 | 1250 | 2102 | 4166 | 3662 | 3146 | 2644 | 2124 | 982 | 1307 | 774 | 542 |
| 37 | 1551 | 1476 | 5408 | 4823 | 1273 | 2136 | 4167 | 3663 | 3147 | 2646 | 2125 | 986 | 1308 | 776 | 543 |
| 38 | 1567 | 1477 | 5412 | 4824 | 1319 | 2153 | 4168 | 3664 | 3148 | 2647 | 2126 | 994 | 1310 | 777 | 544 |
| 39 | 1683 | 1478 | 5413 | 4825 | 1365 | 2182 | 4169 | 3665 | 3149 | 2648 | 2127 | 995 | 1311 | 778 | 545 |
| 40 | 1694 | 1479 | 5414 | 4827 | 1406 | 2208 | 4170 | 3666 | 3150 | 2649 | 2128 | 1002 | 1312 | 779 | 546 |
| 41 | 1897 | 1480 | 5415 | 4828 | 1419 | 2268 | 4171 | 3667 | 3151 | 2650 | 2130 | 1006 | 1313 | 780 | 547 |
| 42 | 1926 | 1481 | 5416 | 4829 | 1423 | 2284 | 4173 | 3668 | 3152 | 2651 | 2131 | 1029 | 1314 | 781 | 548 |
| 43 | 2008 | 1482 | 5417 | 4830 | 1529 | 2403 | 4174 | 3669 | 3155 | 2653 | 2132 | 1032 | 1315 | 782 | 549 |
| 44 | 2033 | 1483 | 5418 | 4831 | 1629 | 2436 | 4175 | 3670 | 3156 | 2654 | 2133 | 1037 | 1316 | 783 | 550 |
| 45 | 2179 | 1485 | 5420 | 4832 | 1786 | 2507 | 4176 | 3671 | 3157 | 2655 | 2134 | 1039 | 1317 | 784 | 551 |
| 46 | 2226 | 1486 | 5421 | 4833 | 1825 | 2547 | 4177 | 3672 | 3158 | 2656 | 2135 | 1048 | 1318 | 785 | 552 |
| 47 | 2281 | 1487 | 5423 | 4834 | 1843 | 2548 | 4178 | 3673 | 3159 | 2657 | 2136 | 1053 | 1319 | 787 | 553 |
| 48 | 2291 | 1488 | 5425 | 4836 | 1863 | 2555 | 4179 | 3675 | 3160 | 2658 | 2137 | 1064 | 1320 | 788 | 554 |
| 49 | 2428 | 1489 | 5426 | 4837 | 1895 | 2583 | 4180 | 3676 | 3161 | 2659 | 2138 | 1069 | 1321 | 789 | 555 |
| 50 | 2489 | 1490 | 5427 | 4838 | 1904 | 2646 | 4181 | 3677 | 3162 | 2660 | 2140 | 1089 | 1322 | 790 | 557 |
| 51 | 2523 | 1491 | 5428 | 4839 | 1910 | 2722 | 4182 | 3678 | 3163 | 2661 | 2141 | 1093 | 1323 | 791 | 558 |
| 52 | 2586 | 1492 | 5429 | 4840 | 1925 | 2724 | 4183 | 3679 | 3164 | 2662 | 2142 | 1094 | 1324 | 792 | 559 |
| 53 | 2642 | 1493 | 5430 | 4841 | 1943 | 2729 | 4184 | 3680 | 3165 | 2663 | 2143 | 1097 | 1325 | 793 | 560 |
| 54 | 2740 | 1494 | 5432 | 4842 | 1947 | 2786 | 4185 | 3681 | 3166 | 2664 | 2144 | 1102 | 1326 | 794 | 561 |
| 55 | 2746 | 1495 | 5433 | 4843 | 2065 | 2811 | 4262 | 3682 | 3167 | 2665 | 2145 | 1112 | 1327 | 795 | 562 |
| 57 | 2783 | 1496 | 5434 | 4844 | 2115 | 2852 | 4294 | 3683 | 3168 | 2666 | 2146 | 1120 | 1328 | 796 | 563 |
| 58 | 2801 | 1498 | 5435 | 4845 | 2136 | 2906 | 4326 | 3684 | 3169 | 2667 | 2147 | 1124 | 1329 | 797 | 564 |
| 60 | 2817 | 1499 | 5436 | 4846 | 2148 | 2910 | 4798 | 3685 | 3170 | 2668 | 2148 | 1128 | 1331 | 798 | 565 |
| 61 | 2819 | 1500 | 5437 | 4848 | 2173 | 2912 | 4801 | 3686 | 3171 | 2669 | 2149 | 1139 | 1332 | 799 | 566 |

| 62 | 63 | 64 | 65 | 66 | 67 | 68 | 69 | 70 | 71 | 72 | 73 | 74 | 76 | 77 | 78 | 79 | 81 | 82 | 83 | 84 | 85 | 86 | 87 | 88 | 89 | 90 | 91 | 92 | 93 | 94 | 95 | 96 |
|---|---|---|---|---|---|---|---|---|---|---|---|---|---|---|---|---|---|---|---|---|---|---|---|---|---|---|---|---|---|---|---|---|
| 2849 | 2867 | 2873 | 2937 | 2941 | 2970 | 3001 | 3015 | 3022 | 3143 | 3310 | 3367 | 3376 | 3480 | 3512 | 3522 | 3531 | 3622 | 3665 | 3694 | 3697 | 3727 | 3769 | 3783 | 3793 | 3817 | 3839 | 3857 | 3862 | 3912 | 3914 | 3942 | 3950 |
| 1501 | 1502 | 1503 | 1504 | 1505 | 1506 | 1507 | 1508 | 1509 | 1510 | 1511 | 1512 | 1513 | 1514 | 1515 | 1516 | 1517 | 1518 | 1519 | 1520 | 1521 | 1522 | 1523 | 1524 | 1525 | 1526 | 1527 | 1528 | 1529 | 1530 | 1532 | 1533 | 1534 |
| 5438 | 5439 | 5440 | 5441 | 5442 | 5443 | 5444 | 5445 | 5446 | 5447 | 5448 | 5449 | 5450 | 5452 | 5453 | 5454 | 5455 | 5456 | 5458 | 5459 | 5460 | 5461 | 5462 | 5464 | 5465 | 5466 | 5468 | 5469 | 5470 | 5471 | 5472 | 5473 | 5475 |
| 4850 | 4851 | 4852 | 4853 | 4854 | 4855 | 4856 | 4857 | 4858 | 4861 | 4862 | 4863 | 4864 | 4866 | 4867 | 4868 | 4869 | 4870 | 4871 | 4872 | 4876 | 4877 | 4878 | 4879 | 4880 | 4881 | 4882 | 4883 | 4884 | 4885 | 4887 | 4888 | 4889 |
| 2250 | 2295 | 2407 | 2479 | 2574 | 2596 | 2602 | 2622 | 2676 | 2684 | 2739 | 2782 | 2788 | 2836 | 2932 | 2947 | 3073 | 3172 | 3183 | 3194 | 3220 | 3237 | 3307 | 3434 | 3437 | 3512 | 3590 | 3602 | 3610 | 3803 | 3996 | 4047 | 4100 |
| 2980 | 3002 | 3022 | 3104 | 3139 | 3232 | 3241 | 3375 | 3388 | 3427 | 3512 | 3669 | 3683 | 3695 | 3798 | 3807 | 3819 | 3847 | 3862 | 3982 | 4116 | 4158 | 4186 | 4187 | 4188 | 4189 | 4190 | 4191 | 4192 | 4193 | 4194 | 4195 | 4196 |
| 4802 | 4806 | 4808 | 4811 | 4821 | 4825 | 4834 | 4836 | 4843 | 4847 | 4848 | 4850 | 4864 | 4867 | 4878 | 4887 | 4889 | 4890 | 4894 | 4897 | 4905 | 4914 | 4919 | 4933 | 4937 | 4940 | 4947 | 4950 | 4953 | 4954 | 4968 | 4969 | 4973 |
| 3687 | 3688 | 3689 | 3690 | 3691 | 3692 | 3693 | 3694 | 3695 | 3696 | 3697 | 3698 | 3699 | 3700 | 3701 | 3702 | 3703 | 3704 | 3705 | 3706 | 3707 | 3708 | 3709 | 3710 | 3711 | 3712 | 3713 | 3714 | 3715 | 3716 | 3717 | 3718 | 3719 |
| 3173 | 3174 | 3175 | 3176 | 3177 | 3178 | 3179 | 3180 | 3181 | 3182 | 3183 | 3184 | 3185 | 3186 | 3187 | 3188 | 3189 | 3191 | 3192 | 3193 | 3196 | 3197 | 3198 | 3199 | 3200 | 3201 | 3202 | 3203 | 3204 | 3205 | 3206 | 3207 | 3208 |
| 2670 | 2671 | 2672 | 2673 | 2674 | 2675 | 2676 | 2677 | 2678 | 2679 | 2680 | 2681 | 2682 | 2683 | 2685 | 2686 | 2687 | 2688 | 2689 | 2690 | 2691 | 2692 | 2693 | 2694 | 2696 | 2697 | 2698 | 2699 | 2700 | 2701 | 2702 | 2703 | 2704 |
| 2150 | 2151 | 2152 | 2153 | 2154 | 2156 | 2157 | 2158 | 2159 | 2160 | 2161 | 2163 | 2164 | 2165 | 2167 | 2168 | 2169 | 2170 | 2171 | 2172 | 2174 | 2175 | 2176 | 2177 | 2178 | 2179 | 2180 | 2181 | 2182 | 2183 | 2184 | 2185 | 2186 |
| 1142 | 1171 | 1189 | 1194 | 1199 | 1201 | 1203 | 1212 | 1216 | 1219 | 1228 | 1234 | 1237 | 1238 | 1239 | 1244 | 1248 | 1261 | 1267 | 1281 | 1292 | 1298 | 1308 | 1317 | 1319 | 1334 | 1346 | 1352 | 1364 | 1367 | 1369 | 1384 | 1388 |
| 1334 | 1335 | 1336 | 1338 | 1339 | 1340 | 1341 | 1342 | 1343 | 1344 | 1346 | 1347 | 1348 | 1349 | 1350 | 1351 | 1352 | 1354 | 1355 | 1356 | 1358 | 1359 | 1360 | 1361 | 1362 | 1363 | 1364 | 1366 | 1367 | 1368 | 1369 | 1370 | 1371 |
| 800 | 801 | 802 | 803 | 804 | 806 | 807 | 808 | 809 | 810 | 811 | 812 | 814 | 815 | 817 | 818 | 819 | 821 | 822 | 823 | 824 | 825 | 826 | 827 | 829 | 830 | 831 | 832 | 833 | 834 | 835 | 836 | 837 |
| 567 | 568 | 569 | 570 | 571 | 572 | 573 | 574 | 575 | 576 | 577 | 578 | 580 | 581 | 582 | 583 | 584 | 585 | 586 | 587 | 588 | 589 | 590 | 591 | 592 | 593 | 594 | 595 | 597 | 598 | 599 | 600 | 601 |

| | | | | | | | | | | | | | | | |
|---|---|---|---|---|---|---|---|---|---|---|---|---|---|---|---|
| 3994 | 1535 | 5477 | 4890 | 4101 | 4197 | 4975 | 3720 | 3210 | 2705 | 2187 | 1390 | 1372 | 838 | 602 | 97 |
| 4028 | 1536 | 5478 | 4891 | 4646 | 4198 | 4987 | 3721 | 3211 | 2706 | 2188 | 1408 | 1373 | 840 | 603 | 98 |
| 4066 | 1537 | 5479 | 4892 | 4813 | 4199 | 4992 | 3722 | 3212 | 2707 | 2189 | 1410 | 1375 | 841 | 604 | 99 |
| 4303 | 1538 | 5480 | 4893 | 4847 | 4200 | 4993 | 3723 | 3213 | 2708 | 2190 | 1413 | 1376 | 842 | 605 | 100 |
| 4326 | 1539 | 5482 | 4894 | 4873 | 4201 | 4998 | 3724 | 3214 | 2709 | 2191 | 1415 | 1377 | 844 | 606 | 101 |
| 4329 | 1540 | 5483 | 4895 | 4875 | 4202 | 5003 | 3725 | 3215 | 2710 | 2192 | 1423 | 1378 | 845 | 607 | 102 |
| 4384 | 1541 | 5484 | 4896 | 4950 | 4203 | 5005 | 3726 | 3216 | 2711 | 2193 | 1467 | 1379 | 846 | 608 | 103 |
| 4503 | 1542 | 5485 | 4897 | 4996 | 4204 | 5009 | 3727 | 3218 | 2712 | 2194 | 1478 | 1380 | 847 | 609 | 104 |
| 4504 | 1543 | 5486 | 4898 | 5014 | 4205 | 5010 | 3728 | 3219 | 2713 | 2195 | 1483 | 1381 | 848 | 610 | 105 |
| 4505 | 1544 | 5487 | 4899 | 5035 | 4206 | 5014 | 3729 | 3220 | 2714 | 2196 | 1494 | 1382 | 849 | 611 | 106 |
| 4506 | 1545 | 5488 | 4900 | 5037 | 4207 | 5021 | 3730 | 3221 | 2715 | 2197 | 1500 | 1383 | 852 | 612 | 107 |
| 4507 | 1546 | 5489 | 4902 | 5087 | 4208 | 5026 | 3731 | 3222 | 2716 | 2199 | 1503 | 1384 | 853 | 613 | 108 |
| 4508 | 1547 | 5490 | 4904 | 5113 | 4209 | 5040 | 3732 | 3223 | 2717 | 2200 | 1508 | 1385 | 854 | 614 | 109 |
| 4509 | 1548 | 5492 | 4905 | 5138 | 4210 | 5043 | 3733 | 3225 | 2718 | 2202 | 1515 | 1386 | 855 | 615 | 110 |
| 4510 | 1549 | 5493 | 4906 | 5140 | 4211 | 5048 | 3734 | 3226 | 2719 | 2203 | 1527 | 1387 | 856 | 616 | 111 |
| 4511 | 1550 | 5494 | 4907 | 5141 | 4212 | 5056 | 3735 | 3227 | 2720 | 2204 | 1533 | 1388 | 857 | 617 | 112 |
| 4512 | 1551 | 5496 | 4908 | 5178 | 4213 | 5064 | 3736 | 3228 | 2721 | 2205 | 1535 | 1389 | 858 | 618 | 113 |
| 4513 | 1552 | 5497 | 4909 | 5181 | 4214 | 5066 | 3737 | 3229 | 2722 | 2206 | 1542 | 1390 | 859 | 619 | 114 |
| 4514 | 1553 | 5498 | 4910 | 5304 | 4215 | 5071 | 3738 | 3230 | 2723 | 2207 | 1550 | 1391 | 860 | 620 | 116 |
| 4515 | 1554 | 5499 | 4911 | 5323 | 4216 | 5073 | 3739 | 3231 | 2724 | 2208 | 1552 | 1392 | 861 | 621 | 117 |
| 4516 | 1555 | 5500 | 4913 | 5346 | 4217 | 5075 | 3740 | 3232 | 2725 | 2209 | 1559 | 1393 | 862 | 622 | 118 |
| 4517 | 1556 | 5501 | 4914 | 5349 | 4218 | 5077 | 3741 | 3233 | 2726 | 2210 | 1565 | 1394 | 863 | 624 | 119 |
| 4518 | 1557 | 5502 | 4915 | 5350 | 4219 | 5080 | 3742 | 3234 | 2727 | 2211 | 1576 | 1395 | 864 | 625 | 120 |
| 4519 | 1558 | 5503 | 4916 | 5354 | 4220 | 5083 | 3743 | 3235 | 2728 | 2212 | 1579 | 1396 | 865 | 626 | 121 |
| 4520 | 1559 | 5504 | 4917 | 5362 | 4221 | 5087 | 3744 | 3236 | 2729 | 2213 | 1580 | 1397 | 866 | 628 | 122 |
| 4521 | 1560 | 5505 | 4918 | 5587 | 4222 | 5088 | 3745 | 3237 | 2730 | 2214 | 1581 | 1398 | 867 | 630 | 123 |
| 4522 | 1561 | 5506 | 4919 | 5597 | 4223 | 5092 | 3746 | 3238 | 2731 | 2215 | 1589 | 1399 | 868 | 631 | 124 |
| 4523 | 1562 | 5507 | 4920 | 5645 | 4224 | 5093 | 3747 | 3239 | 2732 | 2216 | 1604 | 1400 | 869 | 632 | 125 |
| 4524 | 1563 | 5508 | 4921 | 5668 | 4225 | 5097 | 3748 | 3240 | 2733 | 2217 | 1615 | 1402 | 870 | 633 | 126 |
| 4525 | 1564 | 5509 | 4922 | 5716 | 4226 | 5099 | 3749 | 3241 | 2734 | 2218 | 1624 | 1403 | 871 | 634 | 127 |
| 4526 | 1565 | 5511 | 4923 | 5717 | 4227 | 5102 | 3750 | 3242 | 2735 | 2219 | 1629 | 1404 | 872 | 635 | 128 |
| 4527 | 1566 | 5512 | 4924 | 5718 | 4228 | 5103 | 3751 | 3243 | 2736 | 2220 | 1644 | 1405 | 873 | 636 | 129 |
| 4528 | 1567 | 5513 | 4925 | 5719 | 4229 | 5112 | 3752 | 3244 | 2737 | 2221 | 1645 | 1407 | 874 | 637 | 130 |

| | | | | | | | | | | | | | | | |
|---|---|---|---|---|---|---|---|---|---|---|---|---|---|---|---|
| 4529 | 1568 | 5514 | 4926 | 5720 | 4230 | 5113 | 3753 | 3245 | 2738 | 2222 | 1646 | 1408 | 875 | 647 | 131 |
| 4530 | 1569 | 5515 | 4927 | 5721 | 4231 | 5115 | 3754 | 3246 | 2739 | 2223 | 1651 | 1409 | 876 | 648 | 132 |
| 4531 | 1570 | 5517 | 4928 | 5722 | 4232 | 5119 | 3755 | 3248 | 2740 | 2224 | 1652 | 1410 | 877 | 649 | 133 |
| 4532 | 1571 | 5518 | 4929 | 5723 | 4233 | 5124 | 3756 | 3249 | 2741 | 2225 | 1665 | 1411 | 878 | 650 | 134 |
| 4533 | 1572 | 5519 | 4930 | 5724 | 4234 | 5127 | 3757 | 3250 | 2742 | 2226 | 1693 | 1412 | 879 | 651 | 135 |
| 4534 | 1574 | 5520 | 4931 | 5725 | 4235 | 5128 | 3758 | 3251 | 2743 | 2227 | 1696 | 1413 | 880 | 652 | 136 |
| 4535 | 1575 | 5521 | 4932 | 5726 | 4236 | 5129 | 3759 | 3252 | 2744 | 2228 | 1716 | 1414 | 881 | 653 | 137 |
| 4536 | 1576 | 5522 | 4933 | 5727 | 4237 | 5143 | 3760 | 3254 | 2745 | 2229 | 1717 | 1415 | 882 | 654 | 138 |
| 4537 | 1577 | 5523 | 4934 | 5728 | 4238 | 5148 | 3761 | 3255 | 2746 | 2230 | 1718 | 1416 | 884 | 655 | 139 |
| 4538 | 1578 | 5524 | 4935 | 5729 | 4239 | 5164 | 3762 | 3256 | 2747 | 2231 | 1719 | 1417 | 886 | 656 | 140 |
| 4539 | 1579 | 5525 | 4936 | 5730 | 4240 | 5165 | 3763 | 3257 | 2748 | 2232 | 1720 | 1418 | 887 | 657 | 142 |
| 4540 | 1580 | 5526 | 4937 | 5731 | 4241 | 5168 | 3764 | 3258 | 2749 | 2233 | 1721 | 1419 | 888 | 658 | 143 |
| 4541 | 1581 | 5527 | 4938 | 5732 | 4242 | 5179 | 3765 | 3259 | 2750 | 2234 | 1722 | 1420 | 889 | 659 | 144 |
| 4542 | 1582 | 5528 | 4939 | 5733 | 4243 | 5190 | 3766 | 3260 | 2751 | 2235 | 1723 | 1421 | 890 | 660 | 145 |
| 4543 | 1583 | 5529 | 4940 | 5734 | 4244 | 5192 | 3767 | 3261 | 2752 | 2236 | 1724 | 1422 | 891 | 661 | 146 |
| 4544 | 1584 | 5530 | 4943 | 5735 | 4245 | 5196 | 3768 | 3262 | 2753 | 2237 | 1725 | 1423 | 892 | 662 | 147 |
| 4545 | 1585 | 5531 | 4945 | 5736 | 4246 | 5197 | 3769 | 3263 | 2755 | 2239 | 1726 | 1424 | 893 | 663 | 148 |
| 4546 | 1586 | 5532 | 4946 | 5737 | 4247 | 5198 | 3770 | 3264 | 2756 | 2240 | 1727 | 1425 | 894 | 664 | 149 |
| 4547 | 1587 | 5533 | 4947 | 5738 | 4248 | 5203 | 3771 | 3265 | 2757 | 2241 | 1728 | 1426 | 895 | 665 | 150 |
| 4548 | 1588 | 5534 | 4950 | 5739 | 4249 | 5208 | 3772 | 3266 | 2758 | 2242 | 1729 | 1427 | 896 | 666 | 151 |
| 4549 | 1589 | 5535 | 4951 | 5740 | 4250 | 5214 | 3773 | 3267 | 2759 | 2243 | 1730 | 1428 | 897 | 667 | 152 |
| 4550 | 1590 | 5536 | 4953 | 5741 | 4251 | 5218 | 3774 | 3268 | 2760 | 2244 | 1731 | 1429 | 898 | 668 | 153 |
| 4551 | 1591 | 5537 | 4954 | 5742 | 4252 | 5224 | 3775 | 3269 | 2761 | 2245 | 1732 | 1430 | 899 | 638 | 154 |
| 4552 | 1592 | 5538 | 4955 | 5743 | 4253 | 5228 | 3776 | 3270 | 2762 | 2246 | 1733 | 1431 | 900 | 640 | 155 |
| 4553 | 1593 | 5539 | 4956 | 5744 | 4254 | 5231 | 3777 | 3271 | 2763 | 2247 | 1734 | 1432 | 901 | 641 | 156 |
| 4554 | 1594 | 5540 | 4957 | 5745 | 4255 | 5244 | 3778 | 3272 | 2764 | 2248 | 1735 | 1433 | 903 | 642 | 157 |
| 4555 | 1595 | 5541 | 4959 | 5746 | 4256 | 5246 | 3779 | 3273 | 2765 | 2249 | 1736 | 1434 | 904 | 643 | 158 |
| 4556 | 1596 | 5542 | 4960 | 5747 | 4257 | 5247 | 3780 | 3274 | 2766 | 2250 | 1737 | 1435 | 905 | 644 | 160 |
| 4557 | 1597 | 5543 | 4961 | 5748 | 4258 | 5251 | 3781 | 3275 | 2767 | 2251 | 1738 | 1436 | 906 | 645 | 161 |
| 4558 | 1598 | 5544 | 4963 | 5749 | 4259 | 5270 | 3782 | 3276 | 2768 | 2253 | 1739 | 1437 | 907 | 646 | 162 |
| 4559 | 1599 | 5545 | 4966 | 5750 | 4260 | 5272 | 3783 | 3277 | 2769 | 2254 | 1740 | 1438 | 909 | 675 | 163 |
| 4560 | 1600 | 5546 | 4967 | 5751 | 4261 | 5277 | 3784 | 3279 | 2770 | 2255 | 1742 | 1439 | 910 | 676 | 164 |
| 4561 | 1601 | 5547 | 4968 | 5752 | 4262 | 5281 | 3785 | 3280 | 2771 | 2256 | 1743 | 1440 | 911 | 680 | 165 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 4562 | 4563 | 4564 | 4565 | 4566 | 4567 | 4568 | 4569 | 4570 | 4571 | 4572 | 4573 | 4574 | 4575 | 4576 | 4577 | 4578 | 4579 | 4580 | 4581 | 4582 | 4583 | 4584 | 4585 | 4586 | 4587 | 4588 | 4589 | 4590 | 4591 | 4592 | 4593 | 4594 |
| 1602 | 1603 | 1604 | 1605 | 1606 | 1607 | 1608 | 1609 | 1610 | 1611 | 1612 | 1613 | 1614 | 1615 | 1616 | 1617 | 1618 | 1619 | 1620 | 1621 | 1622 | 1623 | 1624 | 1625 | 1626 | 1628 | 1629 | 1630 | 1632 | 1633 | 1634 | 1635 | 1636 |
| 5548 | 5549 | 5551 | 5552 | 5554 | 5555 | 5556 | 5557 | 5558 | 5560 | 5561 | 5562 | 5563 | 5564 | 5565 | 5566 | 5567 | 5568 | 5569 | 5570 | 5571 | 5572 | 5573 | 5574 | 5575 | 5576 | 5577 | 5578 | 5580 | 5581 | 5582 | 5583 | 5584 |
| 4969 | 4971 | 4972 | 4973 | 4974 | 4975 | 4976 | 4977 | 4978 | 4979 | 4981 | 4982 | 4985 | 4986 | 4987 | 4989 | 4992 | 4993 | 4994 | 4995 | 4998 | 4999 | 5000 | 5001 | 5002 | 5003 | 5004 | 5005 | 5007 | 5008 | 5009 | 5010 | 5011 |
| 5753 | 5754 | 5755 | 5756 | 5757 | 5758 | 5759 | 5760 | 5761 | 5762 | 5763 | 5764 | 5765 | 5766 | 5767 | 5768 | 5769 | 5770 | 5771 | 5772 | 5773 | 5774 | 5775 | 5776 | 5777 | 5778 | 5779 | 5780 | 5781 | 5782 | 5783 | 5784 | 5785 |
| 4263 | 4264 | 4265 | 4266 | 4267 | 4268 | 4269 | 4270 | 4271 | 4272 | 4273 | 4274 | 4275 | 4276 | 4277 | 4278 | 4279 | 4280 | 4281 | 4282 | 4283 | 4284 | 4285 | 4286 | 4287 | 4288 | 4289 | 4290 | 4291 | 4292 | 4293 | 4294 | 4295 |
| 5291 | 5293 | 5294 | 5310 | 5316 | 5318 | 5329 | 5335 | 5337 | 5341 | 5344 | 5346 | 5348 | 5350 | 5357 | 5366 | 5374 | 5382 | 5383 | 5390 | 5393 | 5403 | 5406 | 5408 | 5416 | 5417 | 5433 | 5435 | 5436 | 5438 | 5439 | 5440 | 5443 |
| 3786 | 3787 | 3788 | 3789 | 3790 | 3791 | 3792 | 3793 | 3794 | 3795 | 3796 | 3797 | 3798 | 3799 | 3800 | 3801 | 3802 | 3803 | 3804 | 3805 | 3806 | 3807 | 3809 | 3810 | 3811 | 3812 | 3813 | 3814 | 3815 | 3816 | 3819 | 3820 | 3821 |
| 3281 | 3282 | 3283 | 3285 | 3286 | 3287 | 3288 | 3289 | 3290 | 3291 | 3292 | 3293 | 3294 | 3295 | 3296 | 3297 | 3298 | 3299 | 3300 | 3301 | 3302 | 3303 | 3304 | 3305 | 3306 | 3308 | 3309 | 3310 | 3311 | 3312 | 3313 | 3314 | 3315 |
| 2772 | 2773 | 2774 | 2775 | 2776 | 2777 | 2778 | 2779 | 2780 | 2781 | 2783 | 2784 | 2785 | 2786 | 2787 | 2788 | 2789 | 2790 | 2791 | 2793 | 2794 | 2795 | 2796 | 2797 | 2798 | 2799 | 2800 | 2801 | 2802 | 2803 | 2804 | 2805 | 2806 |
| 2257 | 2258 | 2259 | 2260 | 2261 | 2262 | 2263 | 2264 | 2265 | 2266 | 2267 | 2268 | 2269 | 2270 | 2271 | 2273 | 2274 | 2275 | 2276 | 2277 | 2278 | 2279 | 2280 | 2281 | 2282 | 2283 | 2284 | 2285 | 2286 | 2287 | 2288 | 2290 | 2291 |
| 1744 | 1745 | 1746 | 1747 | 1748 | 1749 | 1750 | 1751 | 1752 | 1753 | 1754 | 1755 | 1757 | 1758 | 1759 | 1760 | 1761 | 1762 | 1763 | 1764 | 1765 | 1766 | 1767 | 1768 | 1769 | 1770 | 1771 | 1772 | 1773 | 1774 | 1775 | 1776 | 1777 |
| 1441 | 1442 | 1443 | 1444 | 1445 | 1446 | 1447 | 1448 | 1449 | 1450 | 1451 | 1452 | 1453 | 1454 | 1455 | 1456 | 1457 | 1458 | 1459 | 1460 | 1461 | 1462 | 1463 | 1464 | 1500 | 1535 | 1559 | 1565 | 1576 | 1622 | 1624 | 1645 | 1651 |
| 912 | 914 | 915 | 916 | 917 | 918 | 919 | 920 | 921 | 922 | 926 | 927 | 928 | 929 | 930 | 931 | 932 | 933 | 934 | 935 | 936 | 937 | 938 | 939 | 940 | 941 | 942 | 943 | 944 | 945 | 946 | 947 | 948 |
| 681 | 682 | 684 | 685 | 686 | 689 | 690 | 691 | 692 | 693 | 694 | 695 | 696 | 697 | 698 | 699 | 700 | 701 | 702 | 703 | 704 | 705 | 706 | 708 | 709 | 710 | 711 | 712 | 713 | 714 | 715 | 716 | 717 |
| 166 | 167 | 168 | 169 | 170 | 171 | 172 | 173 | 174 | 175 | 176 | 177 | 178 | 179 | 180 | 181 | 182 | 183 | 184 | 185 | 186 | 187 | 188 | 189 | 190 | 191 | 192 | 193 | 194 | 195 | 196 | 197 | 198 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 4595 | 4596 | 4597 | 4598 | 4599 | 4600 | 4601 | 4602 | 4603 | 4604 | 4605 | 4606 | 4607 | 4608 | 4609 | 4610 | 4611 | 4612 | 4613 | 4614 | 4615 | 4616 | 4617 | 4618 | 4619 | 4620 | 4621 | 4622 | 4623 | 4624 | 4625 | 4626 | 4627 |
| 1637 | 1639 | 1640 | 1641 | 1642 | 1643 | 1644 | 1645 | 1646 | 1647 | 1648 | 1649 | 1650 | 1651 | 1652 | 1653 | 1654 | 1655 | 1656 | 1657 | 1658 | 1659 | 1660 | 1661 | 1662 | 1663 | 1664 | 1665 | 1666 | 1667 | 1668 | 1669 | 1670 |
| 5588 | 5589 | 5590 | 5591 | 5592 | 5593 | 5594 | 5595 | 5596 | 5599 | 5600 | 5601 | 5602 | 5603 | 5604 | 5605 | 5606 | 5608 | 5610 | 5611 | 5612 | 5613 | 5614 | 5615 | 5617 | 5618 | 5619 | 5620 | 5624 | 5626 | 5627 | 5628 | 5629 |
| 5012 | 5015 | 5017 | 5018 | 5019 | 5020 | 5021 | 5022 | 5025 | 5026 | 5027 | 5028 | 5029 | 5030 | 5031 | 5032 | 5033 | 5034 | 5036 | 5038 | 5039 | 5040 | 5041 | 5042 | 5043 | 5044 | 5046 | 5047 | 5048 | 5049 | 5050 | 5051 | 5052 |
| 5787 | 5788 | 5789 | 5790 | 5791 | 5792 | 5793 | 5794 | 5795 | 5796 | 5797 | 5798 | 5799 | 5800 | 5801 | 5802 | 5803 | 5804 | 5805 | 5806 | 5807 | 5808 | 5809 | 5810 | 5811 | 5812 | 5813 | 5814 | 5815 | 5816 | 5817 | 5818 | 5819 |
| 4296 | 4297 | 4298 | 4299 | 4300 | 4301 | 4302 | 4303 | 4304 | 4305 | 4306 | 4307 | 4308 | 4309 | 4310 | 4311 | 4312 | 4313 | 4314 | 4315 | 4316 | 4317 | 4318 | 4319 | 4320 | 4321 | 4322 | 4323 | 4324 | 4325 | 4326 | 4327 | 4328 |
| 5444 | 5445 | 5449 | 5450 | 5453 | 5458 | 5464 | 5471 | 5472 | 5478 | 5479 | 5488 | 5489 | 5498 | 5499 | 5500 | 5504 | 5513 | 5514 | 5519 | 5525 | 5526 | 5529 | 5544 | 5549 | 5551 | 5562 | 5563 | 5564 | 5570 | 5573 | 5576 | 5582 |
| 3822 | 3823 | 3825 | 3826 | 3827 | 3828 | 3829 | 3831 | 3832 | 3833 | 3834 | 3835 | 3836 | 3837 | 3838 | 3839 | 3840 | 3841 | 3842 | 3843 | 3844 | 3845 | 3846 | 3847 | 3848 | 3849 | 3850 | 3851 | 3852 | 3853 | 3854 | 3855 | 3856 |
| 3316 | 3317 | 3318 | 3319 | 3320 | 3321 | 3322 | 3323 | 3324 | 3325 | 3326 | 3327 | 3329 | 3330 | 3331 | 3332 | 3333 | 3334 | 3335 | 3338 | 3339 | 3340 | 3341 | 3342 | 3343 | 3344 | 3345 | 3346 | 3347 | 3348 | 3349 | 3350 | 3351 |
| 2808 | 2809 | 2810 | 2811 | 2812 | 2813 | 2814 | 2815 | 2816 | 2817 | 2818 | 2819 | 2820 | 2821 | 2822 | 2823 | 2824 | 2825 | 2826 | 2828 | 2829 | 2830 | 2831 | 2832 | 2833 | 2834 | 2835 | 2837 | 2838 | 2839 | 2840 | 2841 | 2842 |
| 2292 | 2293 | 2294 | 2295 | 2296 | 2297 | 2298 | 2299 | 2300 | 2301 | 2302 | 2303 | 2304 | 2305 | 2306 | 2307 | 2308 | 2309 | 2310 | 2311 | 2312 | 2313 | 2314 | 2315 | 2316 | 2317 | 2318 | 2319 | 2320 | 2321 | 2322 | 2323 | 2324 |
| 1778 | 1779 | 1780 | 1781 | 1782 | 1783 | 1784 | 1785 | 1786 | 1787 | 1788 | 1789 | 1790 | 1791 | 1792 | 1793 | 1794 | 1795 | 1796 | 1797 | 1798 | 1799 | 1800 | 1801 | 1803 | 1804 | 1805 | 1806 | 1807 | 1808 | 1809 | 1810 | 1811 |
| 1709 | 1721 | 1764 | 1792 | 1795 | 1827 | 1875 | 1880 | 1925 | 1926 | 2039 | 2103 | 2115 | 2206 | 2221 | 2231 | 2233 | 2305 | 2307 | 2323 | 2329 | 2344 | 2365 | 2375 | 2422 | 2424 | 2443 | 2449 | 2453 | 2455 | 2456 | 2461 | 2492 |
| 949 | 950 | 951 | 952 | 953 | 954 | 955 | 956 | 957 | 958 | 959 | 960 | 962 | 963 | 964 | 965 | 966 | 967 | 968 | 969 | 971 | 972 | 973 | 974 | 975 | 977 | 978 | 979 | 980 | 981 | 982 | 983 | 984 |
| 719 | 720 | 721 | 722 | 723 | 724 | 725 | 726 | 728 | 729 | 730 | 731 | 732 | 733 | 734 | 735 | 736 | 737 | 738 | 739 | 761 | 775 | 805 | 813 | 816 | 820 | 839 | 843 | 850 | 851 | 885 | 902 | 908 |
| 199 | 200 | 201 | 202 | 203 | 204 | 205 | 206 | 207 | 208 | 209 | 210 | 211 | 212 | 213 | 214 | 215 | 216 | 217 | 218 | 219 | 220 | 221 | 222 | 223 | 224 | 225 | 226 | 227 | 229 | 230 | 231 | 232 |

| 4628 | 4629 | 4630 | 4631 | 4632 | 4633 | 4634 | 4635 | 4636 | 4637 | 4638 | 4639 | 4640 | 4641 | 4642 | 4643 | 4644 | 4645 | 4647 | 4648 | 4649 | 4650 | 4651 | 4652 | 4653 | 4654 | 4655 | 4656 | 4657 | 4658 | 4659 | 4660 | 4661 |
|---|---|---|---|---|---|---|---|---|---|---|---|---|---|---|---|---|---|---|---|---|---|---|---|---|---|---|---|---|---|---|---|---|
| 1671 | 1672 | 1673 | 1674 | 1675 | 1676 | 1677 | 1678 | 1679 | 1680 | 1681 | 1682 | 1683 | 1684 | 1685 | 1686 | 1687 | 1688 | 1689 | 1690 | 1691 | 1692 | 1693 | 1694 | 1695 | 1696 | 1697 | 1698 | 1699 | 1700 | 1701 | 1702 | 1703 |
| 5630 | 5631 | 5632 | 5633 | 5634 | 5635 | 5636 | 5637 | 5638 | 5639 | 5640 | 5643 | 5644 | 5646 | 5647 | 5648 | 5649 | 5650 | 5651 | 5652 | 5653 | 5654 | 5655 | 5656 | 5657 | 5658 | 5659 | 5660 | 5661 | 5662 | 5663 | 5664 | 5665 |
| 5054 | 5055 | 5056 | 5057 | 5058 | 5059 | 5060 | 5063 | 5064 | 5065 | 5066 | 5068 | 5069 | 5071 | 5072 | 5073 | 5075 | 5076 | 5077 | 5078 | 5079 | 5080 | 5081 | 5083 | 5084 | 5088 | 5089 | 5091 | 5092 | 5093 | 5095 | 5097 | 5098 |
| 5820 | 5821 | 5822 | 5823 | 5824 | 5825 | 5826 | 5827 | 5828 | 5829 | 5830 | 5831 | 5832 | 5833 | 5834 | 5835 | 5836 | 5837 | 5838 | 5839 | 5840 | 5841 | 5842 | 5843 | 5844 | 5845 | 5846 | 5847 | 5848 | 5849 | 5850 | 5851 | 5852 |
| 4329 | 4330 | 4331 | 4332 | 4333 | 4334 | 4335 | 4336 | 4337 | 4338 | 4339 | 4340 | 4341 | 4342 | 4343 | 4344 | 4345 | 4346 | 4347 | 4348 | 4349 | 4350 | 4351 | 4352 | 4353 | 4354 | 4355 | 4356 | 4357 | 4358 | 4359 | 4360 | 4361 |
| 5590 | 5602 | 5604 | 5608 | 5617 | 5619 | 5624 | 5628 | 5629 | 5633 | 5635 | 5639 | 5642 | 5645 | 5668 | 5670 | 5671 | 5672 | 5685 | 5694 | 5697 | 5701 | 5703 | 5705 | 5707 | 5708 | 5709 | 5781 | 5838 | 5863 |  |  |  |
| 3857 | 3858 | 3859 | 3860 | 3861 | 3862 | 3863 | 3864 | 3865 | 3866 | 3867 | 3868 | 3869 | 3870 | 3871 | 3872 | 3874 | 3875 | 3876 | 3877 | 3878 | 3879 | 3880 | 3881 | 3882 | 3883 | 3885 | 3886 | 3887 | 3888 | 3889 | 3890 | 3891 |
| 3352 | 3353 | 3354 | 3355 | 3356 | 3357 | 3358 | 3359 | 3360 | 3361 | 3362 | 3363 | 3364 | 3365 | 3366 | 3367 | 3368 | 3369 | 3370 | 3371 | 3372 | 3373 | 3374 | 3375 | 3376 | 3377 | 3378 | 3380 | 3382 | 3383 | 3384 | 3385 | 3386 |
| 2843 | 2844 | 2845 | 2846 | 2847 | 2848 | 2849 | 2850 | 2851 | 2852 | 2853 | 2854 | 2855 | 2856 | 2857 | 2858 | 2859 | 2860 | 2861 | 2862 | 2863 | 2864 | 2865 | 2866 | 2867 | 2868 | 2869 | 2871 | 2872 | 2873 | 2874 | 2875 | 2876 |
| 2326 | 2327 | 2328 | 2329 | 2330 | 2331 | 2332 | 2333 | 2334 | 2335 | 2336 | 2337 | 2338 | 2340 | 2341 | 2342 | 2343 | 2344 | 2345 | 2346 | 2347 | 2348 | 2349 | 2350 | 2351 | 2352 | 2353 | 2354 | 2356 | 2357 | 2358 | 2359 | 2360 |
| 1812 | 1813 | 1814 | 1815 | 1816 | 1817 | 1818 | 1819 | 1820 | 1821 | 1822 | 1823 | 1824 | 1825 | 1826 | 1827 | 1828 | 1829 | 1830 | 1831 | 1832 | 1833 | 1834 | 1835 | 1836 | 1837 | 1839 | 1841 | 1842 | 1844 | 1845 | 1846 | 1847 |
| 2505 | 2512 | 2513 | 2535 | 2573 | 2583 | 2598 | 2638 | 2715 | 2746 | 2753 | 2781 | 2794 | 2810 | 2812 | 2824 | 2837 | 2845 | 2906 | 2986 | 2999 | 3055 | 3109 | 3118 | 3158 | 3165 | 3210 | 3231 | 3239 | 3242 | 3342 | 3346 | 3359 |
| 985 | 986 | 987 | 988 | 990 | 991 | 992 | 993 | 994 | 995 | 996 | 997 | 998 | 999 | 1000 | 1001 | 1002 | 1003 | 1004 | 1005 | 1006 | 1007 | 1008 | 1009 | 1010 | 1011 | 1012 | 1013 | 1015 | 1016 | 1017 | 1018 | 1019 |
| 913 | 923 | 924 | 925 | 961 | 976 | 989 | 1014 | 1036 | 1050 | 1058 | 1062 | 1077 | 1086 | 1091 | 1108 | 1146 | 1148 | 1174 | 1178 | 1197 | 1210 | 1211 | 1233 | 1257 | 1259 | 1263 | 1276 | 1291 | 1309 | 1330 | 1333 | 1337 |
| 233 | 234 | 235 | 236 | 237 | 238 | 239 | 240 | 241 | 242 | 243 | 244 | 245 | 246 | 247 | 248 | 249 | 250 | 251 | 252 | 253 | 254 | 255 | 256 | 257 | 258 | 259 | 260 | 261 | 262 | 263 | 264 | 265 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 4662 | 4663 | 4664 | 4665 | 4666 | 4667 | 4668 | 4669 | 4670 | 4671 | 4672 | 4673 | 4674 | 4675 | 4676 | 4677 | 4678 | 4679 | 4680 | 4681 | 4682 | 4683 | 4684 | 4685 | 4686 | 4687 | 4688 | 4689 | 4690 | 4691 | 4692 | 4693 | 4694 |
| 1704 | 1705 | 1707 | 1709 | 1710 | 1711 | 1712 | 1713 | 1714 | 1715 | 1843 | 1911 | 1917 | 2020 | 2102 | 2141 | 2257 | 2258 | 2281 | 2305 | 2538 | 2567 | 2638 | 2711 | 2794 | 2835 | 2850 | 2958 | 3063 | 3143 | 3231 | 3375 | 3510 |
| 5666 | 5667 | 5668 | 5669 | 5671 | 5672 | 5673 | 5674 | 5675 | 5676 | 5677 | 5678 | 5679 | 5680 | 5681 | 5682 | 5683 | 5684 | 5685 | 5686 | 5688 | 5689 | 5690 | 5691 | 5692 | 5694 | 5695 | 5696 | 5697 | 5698 | 5700 | 5701 | 5702 |
| 5099 | 5100 | 5101 | 5102 | 5103 | 5104 | 5105 | 5106 | 5107 | 5108 | 5110 | 5111 | 5112 | 5113 | 5114 | 5115 | 5117 | 5118 | 5119 | 5121 | 5122 | 5123 | 5124 | 5125 | 5126 | 5127 | 5128 | 5129 | 5131 | 5132 | 5133 | 5135 | 5136 |
| 5853 | 5854 | 5855 | 5856 | 5857 | 5858 | 5859 | 5860 | 5861 | 5862 | 5863 | 5864 | 5865 | 5866 | 5867 | 5868 | 5869 | 5870 | 5871 | 5872 | 5873 | 5874 | 5875 | 5876 | 5877 | 5878 | 5879 | 5880 | 5881 | 5882 | 5883 | 5884 | 5885 |
| 4362 | 4363 | 4364 | 4365 | 4366 | 4367 | 4368 | 4369 | 4370 | 4371 | 4372 | 4373 | 4374 | 4375 | 4376 | 4377 | 4378 | 4379 | 4380 | 4381 | 4382 | 4383 | 4384 | 4385 | 4386 | 4387 | 4388 | 4389 | 4390 | 4391 | 4392 | 4393 | 4394 |
| 3892 | 3893 | 3894 | 3895 | 3897 | 3898 | 3899 | 3900 | 3901 | 3902 | 3903 | 3904 | 3905 | 3906 | 3907 | 3908 | 3909 | 3910 | 3911 | 3912 | 3913 | 3914 | 3915 | 3916 | 3917 | 3918 | 3919 | 3920 | 3922 | 3923 | 3924 | 3925 | 3926 |
| 3387 | 3388 | 3389 | 3390 | 3391 | 3392 | 3393 | 3394 | 3395 | 3396 | 3397 | 3398 | 3399 | 3401 | 3402 | 3403 | 3404 | 3405 | 3406 | 3407 | 3408 | 3409 | 3410 | 3411 | 3412 | 3413 | 3414 | 3415 | 3416 | 3417 | 3418 | 3419 | 3420 |
| 2877 | 2878 | 2879 | 2880 | 2881 | 2882 | 2883 | 2884 | 2885 | 2886 | 2887 | 2888 | 2889 | 2890 | 2891 | 2892 | 2894 | 2895 | 2896 | 2897 | 2898 | 2899 | 2900 | 2901 | 2902 | 2903 | 2904 | 2905 | 2906 | 2907 | 2908 | 2909 | 2910 |
| 2361 | 2362 | 2363 | 2364 | 2365 | 2366 | 2367 | 2368 | 2369 | 2370 | 2371 | 2373 | 2374 | 2375 | 2376 | 2377 | 2378 | 2379 | 2380 | 2381 | 2382 | 2383 | 2384 | 2385 | 2386 | 2387 | 2388 | 2389 | 2391 | 2392 | 2393 | 2394 | 2395 |
| 1848 | 1849 | 1850 | 1851 | 1852 | 1853 | 1854 | 1855 | 1856 | 1857 | 1858 | 1859 | 1860 | 1861 | 1862 | 1863 | 1864 | 1865 | 1866 | 1868 | 1869 | 1870 | 1871 | 1872 | 1873 | 1874 | 1875 | 1876 | 1877 | 1878 | 1879 | 1880 | 1882 |
| 3363 | 3371 | 3421 | 3427 | 3447 | 3458 | 3481 | 3498 | 3525 | 3617 | 3622 | 3658 | 3665 | 3745 | 3787 | 3790 | 3796 | 3810 | 3828 | 3835 | 3838 | 3846 | 3888 | 3908 | 3912 | 3929 | 3942 | 3946 | 3949 | 3977 | 3982 | 3987 | 3990 |
| 1020 | 1021 | 1022 | 1023 | 1024 | 1025 | 1026 | 1027 | 1028 | 1029 | 1030 | 1031 | 1032 | 1033 | 1034 | 1035 | 1037 | 1038 | 1039 | 1040 | 1041 | 1042 | 1043 | 1044 | 1045 | 1046 | 1047 | 1048 | 1049 | 1051 | 1052 | 1053 | 1054 |
| 1345 | 1353 | 1357 | 1374 | 1484 | 1497 | 1531 | 1573 | 1631 | 1638 | 1706 | 1708 | 1741 | 1756 | 1802 | 1838 | 1840 | 1867 | 1881 | 1909 | 1924 | 1944 | 1995 | 2038 | 2050 | 2100 | 2107 | 2120 | 2129 | 2139 | 2155 | 2162 | 2198 |
| 266 | 267 | 268 | 269 | 270 | 271 | 272 | 273 | 274 | 275 | 276 | 277 | 278 | 279 | 280 | 281 | 282 | 283 | 284 | 285 | 286 | 288 | 290 | 292 | 293 | 294 | 295 | 296 | 297 | 298 | 299 | 300 | 301 |

| | | | | | |
|---|---|---|---|---|---|
| 4695 | 3647 | 5703 | 5137 | 5886 | 4395 |
| 4696 | 3685 | 5704 | 5139 | 5887 | 4396 |
| 4697 | 3790 | 5705 | 5142 | 5888 | 4397 |
| 4698 | 3862 | 5706 | 5143 | 5889 | 4398 |
| 4699 | 3873 | 5707 | 5144 | 5890 | 4399 |
| 4700 | 3876 | 5708 | 5145 | 5891 | 4400 |
| 4701 | 3879 | 5709 | 5146 | 5892 | 4401 |
| 4702 | 3935 | 5710 | 5147 | 5893 | 4402 |
| 4703 | 3977 | 5711 | 5148 | 5894 | 4403 |
| 4704 | 3996 | 5712 | 5150 | 5895 | 4404 |
| 4705 | 4007 | 5713 | 5151 | 5896 | 4405 |
| 4706 | 4021 | 5714 | 5152 | 5897 | 4406 |
| 4707 | 4088 | | 5155 | 5898 | 4407 |
| 4708 | 4807 | | 5156 | 5899 | 4408 |
| 4709 | 4821 | | 5157 | 5900 | 4409 |
| 4710 | 4870 | | 5158 | 5901 | 4410 |
| 4711 | 4904 | | 5159 | 5902 | 4411 |
| 4712 | 4940 | | 5160 | 5903 | 4412 |
| 4713 | 4947 | | 5161 | 5904 | 4413 |
| 4714 | 4998 | | 5164 | 5905 | 4414 |
| 4715 | 5005 | | 5165 | 5906 | 4415 |
| 4716 | 5010 | | 5167 | 5907 | 4416 |
| 4717 | 5027 | | 5168 | 5908 | 4417 |
| 4718 | 5092 | | 5169 | 5909 | 4418 |
| 4719 | 5093 | | 5170 | 5910 | 4419 |
| 4720 | 5112 | | 5171 | 5911 | 4420 |
| 4721 | 5128 | | 5172 | 5912 | 4421 |
| 4722 | 5131 | | 5173 | 5913 | 4422 |
| 4723 | 5143 | | 5174 | 5914 | 4423 |
| 4724 | 5160 | | 5175 | 5915 | 4424 |
| 4725 | 5179 | | 5176 | 5916 | 4425 |
| 4726 | 5181 | | 5177 | 5917 | 4426 |
| 4727 | 5190 | | 5179 | 5918 | 4427 |

| | | | | | | | | |
|---|---|---|---|---|---|---|---|---|
| 302 | 2238 | 1055 | 3991 | 1883 | 2396 | 2911 | 3421 | 3927 |
| 303 | 2252 | 1057 | 4021 | 1884 | 2397 | 2912 | 3422 | 3929 |
| 304 | 2272 | 1059 | 4027 | 1885 | 2398 | 2913 | 3423 | 3930 |
| 305 | 2289 | 1060 | 4757 | 1886 | 2400 | 2914 | 3424 | 3931 |
| 306 | 2325 | 1061 | 4821 | 1887 | 2401 | 2915 | 3425 | 3932 |
| 307 | 2339 | 1063 | 4824 | 1888 | 2402 | 2916 | 3427 | 3933 |
| 308 | 2390 | 1064 | 4825 | 1889 | 2403 | 2917 | 3428 | 3934 |
| 309 | 2399 | 1065 | 4832 | 1890 | 2404 | 2918 | 3429 | 3935 |
| 310 | 2412 | 1066 | 4847 | 1891 | 2405 | 2919 | 3430 | 3936 |
| 312 | 2427 | 1067 | 4848 | 1892 | 2406 | 2920 | 3431 | 3937 |
| 313 | 2442 | 1088 | 4864 | 1893 | 2408 | 2921 | 3432 | 3938 |
| 314 | 2450 | 1069 | 4867 | 1894 | 2409 | 2922 | 3433 | 3939 |
| 315 | 2483 | 1070 | 4889 | 1896 | 2410 | 2923 | 3434 | 3940 |
| 316 | 2487 | 1071 | 4894 | 1897 | 2411 | 2924 | 3435 | 3941 |
| 317 | 2497 | 1072 | 4923 | 1898 | 2413 | 2925 | 3436 | 3942 |
| 318 | 2516 | 1073 | 4930 | 1899 | 2414 | 2926 | 3438 | 3943 |
| 319 | 2522 | 1074 | 4940 | 1900 | 2415 | 2927 | 3439 | 3944 |
| 320 | 2552 | 1075 | 4944 | 1901 | 2416 | 2928 | 3440 | 3945 |
| 322 | 2568 | 1076 | 4947 | 1902 | 2417 | 2929 | 3441 | 3946 |
| 323 | 2599 | 1078 | 4953 | 1903 | 2418 | 2930 | 3442 | 3948 |
| 324 | 2608 | 1079 | 4968 | 1905 | 2419 | 2931 | 3443 | 3949 |
| 325 | 2614 | 1081 | 4973 | 1906 | 2420 | 2933 | 3444 | 3950 |
| 326 | 2626 | 1082 | 4977 | 1907 | 2421 | 2934 | 3445 | 3952 |
| 327 | 2632 | 1083 | 4982 | 1908 | 2422 | 2935 | 3446 | 3953 |
| 328 | 2643 | 1084 | 4992 | 1911 | 2423 | 2936 | 3447 | 3954 |
| 329 | 2645 | 1085 | 4998 | 1912 | 2424 | 2937 | 3448 | 3955 |
| 330 | 2695 | 1087 | 5003 | 1913 | 2425 | 2939 | 3449 | 3956 |
| 331 | 2754 | 1088 | 5010 | 1914 | 2426 | 2940 | 3451 | 3957 |
| 332 | 2792 | 1089 | 5017 | 1915 | 2428 | 2941 | 3452 | 3958 |
| 333 | 2807 | 1090 | 5019 | 1916 | 2429 | 2942 | 3453 | 3959 |
| 334 | 2870 | 1092 | 5021 | 1918 | 2430 | 2943 | 3455 | 3960 |
| 335 | 2893 | 1093 | 5026 | 1919 | 2431 | 2944 | 3456 | 3961 |
| 336 | 2938 | 1094 | 5035 | 1920 | 2432 | 2945 | 3457 | 3962 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 4728 | 4729 | 4730 | 4732 | 4733 | 4734 | 4735 | 4736 | 4737 | 4738 | 4739 | 4740 | 4741 | 4743 | 4744 | 4745 | 4746 | 4747 | 4748 | 4749 | 4750 | 4751 | 4752 | 4753 | 4754 | 4755 | 4756 | 4757 | 4758 | 4759 | 4760 | 4761 | 4762 |
| 5196 | 5203 | 5234 | 5236 | 5259 | 5288 | 5294 | 5310 | 5332 | 5350 | 5375 | 5395 | 5397 | 5420 | 5437 | 5438 | 5448 | 5460 | 5465 | 5471 | 5475 | 5476 | 5489 | 5499 | 5504 | 5528 | 5573 | 5585 | 5617 | 5635 | 5674 | 5685 | 5687 |
| 5180 | 5181 | 5182 | 5183 | 5184 | 5185 | 5186 | 5188 | 5189 | 5190 | 5191 | 5192 | 5194 | 5195 | 5196 | 5197 | 5198 | 5200 | 5201 | 5202 | 5203 | 5204 | 5207 | 5208 | 5209 | 5210 | 5211 | 5212 | 5213 | 5214 | 5215 | 5216 | 5217 |
| 5919 | 5920 | 5921 | 5922 | 5923 | 5924 | 5925 | 5926 | 5927 | 5928 | 5929 | 5930 | 5931 | 5932 | 5933 | 5934 | 5935 | 5936 | 5937 | 5938 | 5939 | 5940 | 5941 | 5942 | 5943 | 5944 | 5945 | 5946 | 5947 | 5948 | 5949 | 5950 | 5951 |
| 4428 | 4429 | 4430 | 4431 | 4432 | 4433 | 4434 | 4435 | 4436 | 4437 | 4438 | 4439 | 4440 | 4441 | 4442 | 4443 | 4444 | 4445 | 4446 | 4447 | 4448 | 4449 | 4450 | 4451 | 4452 | 4453 | 4454 | 4455 | 4456 | 4457 | 4458 | 4459 | 4460 |
| 3964 | 3965 | 3966 | 3967 | 3968 | 3969 | 3970 | 3971 | 3972 | 3973 | 3974 | 3975 | 3976 | 3977 | 3978 | 3979 | 3980 | 3981 | 3982 | 3983 | 3984 | 3985 | 3986 | 3987 | 3988 | 3989 | 3990 | 3991 | 3992 | 3993 | 3994 | 3995 | 3996 |
| 3458 | 3459 | 3460 | 3461 | 3462 | 3463 | 3464 | 3465 | 3466 | 3467 | 3468 | 3469 | 3470 | 3471 | 3472 | 3473 | 3474 | 3475 | 3476 | 3477 | 3478 | 3479 | 3480 | 3481 | 3483 | 3484 | 3485 | 3486 | 3487 | 3488 | 3489 | 3490 | 3491 |
| 2946 | 2948 | 2949 | 2950 | 2951 | 2952 | 2953 | 2954 | 2955 | 2956 | 2957 | 2958 | 2959 | 2960 | 2961 | 2962 | 2963 | 2964 | 2965 | 2966 | 2967 | 2968 | 2969 | 2970 | 2971 | 2973 | 2974 | 2976 | 2977 | 2978 | 2979 | 2980 | 2981 |
| 2433 | 2434 | 2435 | 2436 | 2437 | 2438 | 2439 | 2440 | 2441 | 2443 | 2444 | 2445 | 2446 | 2447 | 2448 | 2449 | 2451 | 2452 | 2453 | 2454 | 2455 | 2456 | 2457 | 2458 | 2459 | 2460 | 2461 | 2462 | 2463 | 2464 | 2465 | 2466 | 2467 |
| 1921 | 1922 | 1923 | 1925 | 1926 | 1927 | 1928 | 1929 | 1930 | 1931 | 1932 | 1933 | 1934 | 1935 | 1936 | 1937 | 1938 | 1939 | 1940 | 1941 | 1942 | 1945 | 1946 | 1947 | 1948 | 1949 | 1950 | 1951 | 1952 | 1953 | 1954 | 1955 | 1956 |
| 5061 | 5064 | 5066 | 5093 | 5096 | 5108 | 5112 | 5113 | 5115 | 5124 | 5126 | 5128 | 5137 | 5141 | 5143 | 5144 | 5154 | 5160 | 5164 | 5168 | 5170 | 5179 | 5188 | 5203 | 5228 | 5229 | 5236 | 5251 | 5253 | 5268 | 5272 | 5275 | 5281 |
| 1095 | 1096 | 1097 | 1098 | 1099 | 1100 | 1101 | 1102 | 1103 | 1104 | 1105 | 1106 | 1107 | 1109 | 1110 | 1111 | 1112 | 1113 | 1114 | 1115 | 1116 | 1117 | 1118 | 1119 | 1120 | 1122 | 1123 | 1124 | 1125 | 1126 | 1127 | 1128 | 1129 |
| 2972 | 2975 | 2989 | 3009 | 3053 | 3057 | 3075 | 3153 | 3154 | 3190 | 3195 | 3209 | 3217 | 3224 | 3247 | 3253 | 3278 | 3284 | 3336 | 3337 | 3379 | 3381 | 3400 | 3426 | 3450 | 3454 | 3482 | 3520 | 3521 | 3562 | 3578 | 3583 | 3591 |
| 337 | 338 | 339 | 340 | 341 | 342 | 343 | 344 | 345 | 346 | 347 | 348 | 349 | 350 | 351 | 352 | 353 | 354 | 355 | 356 | 357 | 358 | 359 | 360 | 361 | 362 | 363 | 364 | 365 | 366 | 367 | 369 | 370 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 4763 | 4764 | 4765 | 4766 | 4767 | 4768 | 4769 | 4770 | 4771 | 4772 | 4773 | 4774 | 4775 | 4776 | 4777 | 4778 | 4779 | 4780 | 4781 | 4782 | 4783 | 4784 | 4785 | 4786 | 4787 | 4788 | 4789 | 4790 | 4791 | 4792 | 4793 | 4794 | 4798 |
| 5697 | 5708 | | | | | | | | | | | | | | | | | | | | | | | | | | | | | | | |
| 5218 | 5219 | 5220 | 5221 | 5222 | 5224 | 5225 | 5228 | 5229 | 5230 | 5231 | 5232 | 5233 | 5234 | 5236 | 5237 | 5238 | 5239 | 5240 | 5242 | 5244 | 5245 | 5246 | 5247 | 5250 | 5251 | 5252 | 5253 | 5254 | 5255 | 5256 | 5258 | 5260 |
| 5952 | 5953 | 5954 | 5955 | 5956 | 5957 | 5958 | 5959 | 5960 | 5961 | 5962 | 5963 | 5964 | 5965 | 5966 | 5967 | 5968 | 5969 | 5970 | 5971 | 5972 | 5973 | 5974 | 5975 | 5976 | 5977 | 5978 | 5979 | 5980 | 5981 | 5982 | 5983 | 5984 |
| 4461 | 4462 | 4463 | 4464 | 4465 | 4466 | 4467 | 4468 | 4469 | 4470 | 4471 | 4472 | 4473 | 4474 | 4475 | 4476 | 4477 | 4478 | 4479 | 4480 | 4481 | 4482 | 4483 | 4484 | 4485 | 4486 | 4487 | 4488 | 4489 | 4490 | 4491 | 4492 | 4493 |
| 3997 | 3998 | 3999 | 4000 | 4002 | 4003 | 4005 | 4006 | 4007 | 4008 | 4009 | 4010 | 4011 | 4013 | 4014 | 4015 | 4017 | 4018 | 4019 | 4020 | 4021 | 4022 | 4023 | 4024 | 4025 | 4026 | 4027 | 4028 | 4029 | 4031 | 4032 | 4033 | 4034 |
| 3492 | 3493 | 3494 | 3495 | 3496 | 3497 | 3498 | 3499 | 3500 | 3501 | 3502 | 3503 | 3504 | 3505 | 3506 | 3507 | 3508 | 3509 | 3510 | 3511 | 3512 | 3513 | 3514 | 3515 | 3516 | 3517 | 3518 | 3519 | 3522 | 3523 | 3524 | 3525 | 3526 |
| 2982 | 2983 | 2984 | 2985 | 2986 | 2987 | 2988 | 2990 | 2991 | 2992 | 2993 | 2994 | 2995 | 2996 | 2997 | 2998 | 2999 | 3000 | 3001 | 3002 | 3003 | 3004 | 3005 | 3006 | 3007 | 3008 | 3010 | 3011 | 3012 | 3013 | 3014 | 3015 | 3016 |
| 2468 | 2469 | 2470 | 2471 | 2472 | 2473 | 2474 | 2475 | 2476 | 2477 | 2478 | 2480 | 2481 | 2482 | 2484 | 2485 | 2486 | 2488 | 2489 | 2490 | 2491 | 2492 | 2493 | 2494 | 2495 | 2496 | 2498 | 2499 | 2500 | 2501 | 2502 | 2503 | 2504 |
| 1957 | 1958 | 1959 | 1960 | 1961 | 1962 | 1963 | 1964 | 1965 | 1966 | 1967 | 1968 | 1969 | 1970 | 1971 | 1972 | 1973 | 1974 | 1975 | 1976 | 1977 | 1978 | 1979 | 1980 | 1981 | 1982 | 1983 | 1984 | 1985 | 1986 | 1987 | 1988 | 1989 |
| 5291 | 5294 | 5310 | 5313 | 5316 | 5318 | 5319 | 5325 | 5329 | 5341 | 5346 | 5366 | 5381 | 5394 | 5397 | 5408 | 5416 | 5426 | 5436 | 5437 | 5438 | 5440 | 5443 | 5445 | 5447 | 5460 | 5464 | 5465 | 5472 | 5476 | 5489 | 5490 | 5495 |
| 1130 | 1131 | 1132 | 1133 | 1134 | 1135 | 1136 | 1137 | 1138 | 1139 | 1140 | 1141 | 1143 | 1144 | 1145 | 1147 | 1149 | 1150 | 1151 | 1152 | 1153 | 1154 | 1155 | 1156 | 1157 | 1158 | 1159 | 1160 | 1161 | 1162 | 1163 | 1164 | 1165 |
| 3595 | 3650 | 3654 | 3674 | 3808 | 3818 | 3824 | 3830 | 3884 | 3896 | 3921 | 3928 | 3947 | 3951 | 3963 | 4001 | 4004 | 4016 | 4030 | 4048 | 4093 | 4731 | 4742 | 4826 | 4835 | 4859 | 4874 | 4886 | 4901 | 4903 | 4912 | 4941 | 4948 |
| 371 | 372 | 373 | 374 | 375 | 376 | 377 | 378 | 379 | 380 | 381 | 382 | 383 | 384 | 385 | 386 | 387 | 388 | 389 | 390 | 391 | 392 | 393 | 394 | 395 | 396 | 397 | 398 | 399 | 400 | 401 | 402 | 403 |

16

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 4848 | 4850 | 4858 | 4867 | 4872 | 4875 | 4906 | 4907 | 4940 | 4942 | 4944 | 4947 | 4953 | 4972 | 4973 | 4986 | 4991 | 4998 | 5007 | 5010 | 5017 | 5035 | 5056 | 5058 | 5069 | 5071 | 5119 | 5129 | 5143 | 5162 | 5172 | 5186 | 5192 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 5261 | 5262 | 5263 | 5264 | 5265 | 5267 | 5268 | 5269 | 5270 | 5272 | 5273 | 5274 | 5275 | 5276 | 5277 | 5278 | 5280 | 5281 | 5282 | 5283 | 5284 | 5285 | 5286 | 5287 | 5288 | 5289 | 5291 | 5292 | 5293 | 5294 | 5295 | 5296 | 5297 |

| | | | | | | | | | | | | | | | | | | | | | |
|---|---|---|---|---|---|---|---|---|---|---|---|---|---|---|---|---|---|---|---|---|---|
| 5985 | 5986 | 5987 | 5988 | 5989 | 5990 | 5991 | 5992 | 5993 | 5994 | 5995 | 5996 | 5997 | 5998 | 5999 | 6000 | 6001 | 6002 | 6003 | 6004 | 6005 | 6006 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|
| 4494 | 4495 | 4496 | 4497 | 4498 | 4499 | 4500 | 4501 | 4502 | 4582 | 4806 | 4812 | 4821 | 4825 | 4843 | 4853 | 4875 | 4883 | 4887 | 4906 | 4907 | 4922 | 4937 | 4942 | 4953 | 4961 | 4968 | 4973 | 4977 | 4981 | 5003 | 5010 | 5019 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|
| 4035 | 4036 | 4037 | 4038 | 4039 | 4040 | 4041 | 4042 | 4043 | 4044 | 4045 | 4046 | 4049 | 4050 | 4051 | 4052 | 4053 | 4054 | 4055 | 4056 | 4057 | 4058 | 4059 | 4060 | 4061 | 4062 | 4063 | 4064 | 4065 | 4067 | 4068 | 4069 | 4070 |
| 3527 | 3528 | 3529 | 3530 | 3531 | 3532 | 3533 | 3534 | 3535 | 3536 | 3537 | 3538 | 3539 | 3540 | 3541 | 3542 | 3543 | 3544 | 3545 | 3546 | 3547 | 3548 | 3549 | 3550 | 3551 | 3552 | 3553 | 3554 | 3555 | 3556 | 3557 | 3558 | 3559 |
| 3017 | 3018 | 3019 | 3020 | 3021 | 3022 | 3023 | 3024 | 3025 | 3026 | 3027 | 3028 | 3029 | 3030 | 3031 | 3032 | 3033 | 3034 | 3035 | 3036 | 3037 | 3038 | 3039 | 3040 | 3041 | 3042 | 3043 | 3044 | 3045 | 3046 | 3047 | 3048 | 3049 |
| 2505 | 2506 | 2507 | 2508 | 2509 | 2510 | 2511 | 2512 | 2513 | 2514 | 2515 | 2517 | 2518 | 2519 | 2520 | 2521 | 2523 | 2524 | 2525 | 2526 | 2527 | 2528 | 2529 | 2530 | 2531 | 2532 | 2533 | 2534 | 2535 | 2536 | 2537 | 2539 | 2540 |
| 1990 | 1991 | 1992 | 1993 | 1994 | 1996 | 1997 | 1998 | 1999 | 2000 | 2001 | 2002 | 2003 | 2004 | 2005 | 2006 | 2007 | 2008 | 2009 | 2010 | 2011 | 2012 | 2013 | 2014 | 2015 | 2016 | 2017 | 2018 | 2019 | 2020 | 2021 | 2022 | 2023 |

| | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|
| 5500 | 5504 | 5507 | 5514 | 5523 | 5525 | 5535 | 5539 | 5551 | 5563 | 5569 | 5573 | 5577 | 5580 | 5597 | 5600 | 5603 | 5613 | 5617 | 5628 | 5633 | 5634 | 5635 | 5637 | 5671 | 5685 | 5687 | 5701 | 5703 | 5706 | 5708 | 5709 |
| 1166 | 1167 | 1168 | 1169 | 1170 | 1171 | 1172 | 1173 | 1175 | 1176 | 1177 | 1179 | 1180 | 1181 | 1182 | 1183 | 1184 | 1185 | 1186 | 1187 | 1188 | 1189 | 1190 | 1191 | 1192 | 1193 | 1194 | 1195 | 1196 | 1198 | 1199 | 1200 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|
| 4949 | 4952 | 4958 | 4962 | 4965 | 4970 | 4980 | 4984 | 4988 | 4990 | 4997 | 5006 | 5013 | 5016 | 5023 | 5045 | 5053 | 5062 | 5067 | 5070 | 5074 | 5082 | 5085 | 5086 | 5094 | 5109 | 5116 | 5120 | 5130 | 5134 | 5149 | 5153 | 5163 |
| 404 | 406 | 407 | 408 | 409 | 410 | 411 | 412 | 413 | 414 | 415 | 416 | 417 | 418 | 419 | 420 | 421 | 422 | 424 | 425 | 426 | 427 | 428 | 429 | 430 | 432 | 433 | 434 | 436 | 437 | 438 | 439 | 440 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 5196 | 5208 | 5209 | 5240 | 5253 | 5267 | 5269 | 5275 | 5298 | 5303 | 5304 | 5310 | 5316 | 5344 | 5346 | 5350 | 5381 | 5385 | 5401 | 5406 | 5407 | 5416 | 5418 | 5447 | 5449 | 5450 | 5460 | 5465 | 5466 | 5489 | 5491 | 5495 | 5507 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 5298 | 5299 | 5300 | 5301 | 5302 | 5303 | 5304 | 5306 | 5307 | 5308 | 5310 | 5312 | 5313 | 5314 | 5315 | 5316 | 5317 | 5318 | 5319 | 5320 | 5321 | 5322 | 5323 | 5324 | 5325 | 5326 | 5328 | 5329 | 5330 | 5331 | 5332 | 5333 | 5334 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 5056 | 5073 | 5092 | 5101 | 5102 | 5113 | 5143 | 5173 | 5179 | 5187 | 5198 | 5208 | 5211 | 5229 | 5231 | 5236 | 5259 | 5288 | 5303 | 5322 | 5332 | 5350 | 5354 | 5366 | 5373 | 5375 | 5380 | 5386 | 5390 | 5393 | 5406 | 5417 | 5420 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 4071 | 4072 | 4073 | 4074 | 4075 | 4076 | 4077 | 4078 | 4079 | 4080 | 4081 | 4082 | 4083 | 4084 | 4085 | 4086 | 4087 | 4088 | 4089 | 4090 | 4091 | 4092 | 4094 | 4095 | 4096 | 4097 | 4098 | 4099 | 4100 | 4102 | 4103 | 4104 | 4105 |
| 3560 | 3561 | 3563 | 3564 | 3565 | 3566 | 3567 | 3568 | 3569 | 3570 | 3571 | 3572 | 3573 | 3574 | 3575 | 3576 | 3577 | 3579 | 3580 | 3581 | 3582 | 3584 | 3585 | 3586 | 3587 | 3588 | 3589 | 3590 | 3592 | 3593 | 3594 | 3596 | 3597 |
| 3050 | 3051 | 3052 | 3054 | 3055 | 3056 | 3058 | 3059 | 3080 | 3061 | 3062 | 3063 | 3064 | 3065 | 3066 | 3067 | 3068 | 3069 | 3070 | 3071 | 3072 | 3073 | 3074 | 3076 | 3077 | 3078 | 3079 | 3080 | 3081 | 3082 | 3083 | 3084 | 3085 |
| 2541 | 2542 | 2543 | 2544 | 2545 | 2546 | 2547 | 2548 | 2549 | 2550 | 2551 | 2553 | 2554 | 2555 | 2556 | 2557 | 2558 | 2559 | 2560 | 2561 | 2562 | 2563 | 2564 | 2565 | 2566 | 2567 | 2569 | 2570 | 2571 | 2572 | 2573 | 2574 | 2575 |
| 2024 | 2025 | 2026 | 2027 | 2028 | 2029 | 2030 | 2031 | 2032 | 2033 | 2034 | 2035 | 2036 | 2037 | 2039 | 2040 | 2041 | 2042 | 2043 | 2044 | 2045 | 2046 | 2047 | 2048 | 2049 | 2051 | 2052 | 2053 | 2054 | 2055 | 2056 | 2057 | 2058 |

| | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|
| 1202 | 1203 | 1204 | 1205 | 1206 | 1207 | 1208 | 1209 | 1212 | 1213 | 1214 | 1215 | 1217 | 1218 | 1219 | 1220 | 1221 | 1222 | 1223 | 1224 | 1225 | 1226 | 1227 | 1228 | 1229 | 1230 | 1231 | 1232 | 1234 | 1235 | 1236 | 1237 | 1238 |
| 5166 | 5193 | 5205 | 5206 | 5223 | 5226 | 5227 | 5235 | 5241 | 5243 | 5248 | 5249 | 5257 | 5266 | 5271 | 5279 | 5305 | 5309 | 5311 | 5327 | 5343 | 5352 | 5364 | 5367 | 5370 | 5377 | 5396 | 5409 | 5410 | 5419 | 5422 | 5424 | 5431 |
| 441 | 442 | 443 | 444 | 445 | 446 | 447 | 448 | 449 | 450 | 451 | 452 | 453 | 454 | 455 | 456 | 457 | 458 | 460 | 461 | 462 | 463 | 464 | 465 | 466 | 467 | 468 | 469 | 470 | 471 | 472 | 473 | 474 |

| | | | | | | | | | | |
|---|---|---|---|---|---|---|---|---|---|---|
| 475 | 5451 | 1239 | 2059 | 2576 | 3086 | 3598 | 4106 | 5436 | 5335 | 5533 |
| 476 | 5457 | 1241 | 2060 | 2577 | 3087 | 3599 | 4107 | 5441 | 5336 | 5563 |
| 477 | 5463 | 1242 | 2061 | 2578 | 3088 | 3600 | 4108 | 5447 | 5337 | 5596 |
| 478 | 5467 | 1243 | 2062 | 2579 | 3089 | 3601 | 4109 | 5460 | 5338 | 5600 |
| 479 | 5474 | 1244 | 2063 | 2580 | 3090 | 3603 | 4110 | 5476 | 5339 | 5628 |
| 480 | 5481 | 1245 | 2064 | 2581 | 3091 | 3604 | 4111 | 5495 | 5340 | 5635 |
| 481 | 5510 | 1246 | 2066 | 2582 | 3092 | 3605 | 4112 | 5499 | 5341 | 5660 |
| 483 | 5516 | 1247 | 2067 | 2583 | 3093 | 3606 | 4113 | 5500 | 5342 | 5671 |
| 484 | 5550 | 1248 | 2068 | 2584 | 3094 | 3607 | 4114 | 5502 | 5344 | 5674 |
| 485 | 5553 | 1249 | 2069 | 2585 | 3095 | 3608 | 4115 | 5528 | 5345 | 5677 |
| 486 | 5559 | 1251 | 2070 | 2586 | 3096 | 3609 | 4116 | 5563 | 5346 | 5689 |
| 487 | 5579 | 1252 | 2071 | 2587 | 3097 | 3611 | 4117 | 5583 | 5347 | 5706 |
| 488 | 5586 | 1253 | 2072 | 2588 | 3098 | 3612 | 4118 | 5596 | 5348 | |
| 489 | 5607 | 1254 | 2073 | 2589 | 3099 | 3613 | 4119 | 5597 | 5350 | |
| 490 | 5609 | 1255 | 2074 | 2590 | 3100 | 3614 | 4120 | 5628 | 5351 | |
| 491 | 5616 | 1256 | 2075 | 2591 | 3101 | 3615 | 4121 | 5639 | 5353 | |
| 492 | 5621 | 1258 | 2076 | 2592 | 3102 | 3616 | 4122 | 5655 | 5354 | |
| 493 | 5622 | 1260 | 2077 | 2593 | 3103 | 3617 | 4123 | 5671 | 5355 | |
| 494 | 5623 | 1261 | 2078 | 2594 | 3104 | 3618 | 4124 | 5677 | 5356 | |
| 495 | 5641 | 1262 | 2079 | 2595 | 3105 | 3619 | 4125 | 5685 | 5357 | |
| 496 | 5693 | 1264 | 2080 | 2597 | 3106 | 3620 | 4126 | 5687 | 5358 | |
| 497 | 5699 | 1265 | 2081 | 2598 | 3107 | 3621 | 4127 | 5694 | 5359 | |
| 498 | 5786 | 1266 | 2082 | 2600 | 3108 | 3622 | 4128 | 5710 | 5360 | |
| 499 | | 1267 | 2083 | 2601 | 3109 | 3623 | 4129 | 5715 | 5361 | |
| 500 | | 1268 | 2084 | 2602 | 3110 | 3624 | 4130 | | 5363 | |
| 501 | | 1269 | 2085 | 2603 | 3111 | 3625 | 4131 | | 5365 | |

3. Culture conditions for filamentous fungi

The nucleotide sequences of the above DNAs consist of the nucleotide sequences of genes whose expressions are regulated (promoted or suppressed) by one or a combination of the various culture conditions for filamentous fungi. The use of these genes as primers or probes makes it possible to precisely assay in detail the state of the combination of various conditions. In the present invention, examples of various culture conditions include nutrient-rich, nutrient-deficient, solid, early germination, alkaline, high temperature and low temperature culture conditions.

Examples of filamentous fungi to be cultured include microorganisms belonging to the genus *Aspergillus* (including the genus *Emericella*), the genus *Penicillium*, the genus *Humicola*, the genus *Trichoderma*, the genus *Mucor* and the genus *Fusarium* (see the following genera):

the genus *Penicillium*: all the known species classified into the genus *Penicillium*;
the genus *Humicola*: all the known species classified into the genus *Humicola*;
the genus *Trichoderma*: all the known species classified into the genus *Trichoderma*;
the genus *Mucor*: all the known species classified into the genus *Mucor*; and
the genus *Fusarium*: all the known species classified into the genus *Fusarium*.
*Aspergillus*: *Aspergillus oryzae, Aspergillus fumigatus, Aspergillus flavus, Aspergillus sojae, Aspergillus paraciticus, Aspergillus niger, Aspergillus awamori, Aspergillus kawachii, Aspergillus nidulans* and *Emericella nidulans*.

Further, in the present invention, *Aspergillus oryzae* is preferably used. *Aspergillus oryzae* strain RIB40 used for the determination of the nucleotide sequences of the present invention was deposited on March 4, 2002, with the International Patent Organism Depositary, National Institute of Advanced Industrial Science and Technology (Chuo-6, 1-1-1 Higashi, Tsukuba, Ibaraki, Japan) under Accession Number: FERM BP-7935.

(1) Nutrient-rich culture condition

The nutrient-rich culture condition means a culture condition wherein a koji mold can actively grow in a complete medium containing polypeptone, yeast extract or the like, and 0.1% to 5% (preferably 2%) glucose, and having a pH from 5 to 7 (preferably pH 6.3) at a culture temperature ranging from 25°C to 33°C, preferably at 30°C. Under a typical nutrient-rich culture condition, culturing is performed in the following YPD medium at 30 °C for 22 hours.

YPD medium

Yeast extract: 1%
Bacto-pepton: 2%
Adenine sulfate: 0.04%
Glucose: 2%

DNAs expressed by culturing under this condition are represented by SEQ ID NOS. shown in the 1 st row of Table 1.

(2) Nutrient-deficient culture condition

The nutrient-deficient culture condition means a culture condition containing neither protein nor carbon sources such as carbohydrates, wherein a koji mold cannot grow. A koji mold is previously cultured under a culture condition wherein a koji mold can grow, preferably in the above YPD liquid medium. The cells are collected and washed, and then cultured in a medium containing no carbon sources such as glucose with a pH of 5 to 7 (preferably pH 5.5) at 25°C to 33°C (preferably 30°C) for 4 to 10 hours (preferably 8 hours). Under a typical nutrient-deficient culture condition, cells are cultured in the following YPD liquid medium at 30°C for 22 hours, collected by filtration, washed with sterilized water, and then cultured in the following CD (Czapek-Dox) liquid medium at 30°C for 8 hours.

YPD liquid medium

Yeast extract: 1%

Bacto-pepton: 2%
Adenine sulfate: 0.04%
Glucose: 2%

Koji mold cells are cultured under this condition at 30°C for 22 hours, collected, washed, and then transferred to the following nutrient-deficient medium.

CD liquid medium

NaNO$_3$: 0.3%
KCl: 0.2%
KH$_2$PO$_4$: 0.1%
MgSO$_4$·7H$_2$O: 0.05%
FeSO$_4$·7H$_2$O: 0.001%
pH 5.5

DNAs expressed by culturing under this condition are represented by SEQ ID NOS. shown in the 2nd row of Table 1.

(3) Solid culture condition

The solid culture condition means a culture state wherein a koji mold is grown using a medium, for example, grain comprising solid carbohydrates or protein such as steamed rice, wheat and soybeans. A similar culture condition is realized by placing a membrane filter in tight contact with an agar medium, upon which cells are then grown. Under a typical solid culture condition, conidia are adhered to wheat bran, and then cultured at 30°C for 27 hours.
DNAs expressed by culturing under this condition are represented by SEQ ID NOS. shown in the 3rd row of Table 1.

(4) Early germination culture condition

The early germination culture condition means a culture condition immediately after the germination of conidia by culturing at 25°C to 33°C (preferably 30°C) for 8 to 15 hours (preferably 12 hours) following the inoculation of the conidia in a liquid or on an agar medium where a koji mold can grow. Under a typical early germination culture condition, culturing is performed in the following SP liquid medium at 30°C for 12 hours.

SP liquid medium

Soluble starch: 3.5%
Polypeptone: 2%
KH$_2$PO$_4$ : 0.5%
MgSO$_4$·7H$_2$O : 0.5%

DNAs expressed by culturing under this condition are represented by SEQ ID NOS. shown in the 4th row of Table 1.

(5) Alkaline culture condition

The alkaline culture condition means a culture condition wherein koji mold cells grow in an alkaline environment. Specifically, koji mold conidia are inoculated in liquid or on an agar media containing carbon sources and nitrogen sources necessary for the growth of a koji mold and adjusted to have a pH of 8 to 10 (preferably pH 10), and then cultured at 25°C to 33°C (preferably 30°C) for 1 day to 1 week (preferably 3 days). Under a typical alkaline culture condition, a koji mold is cultured in the following CD liquid medium adjusted to have pH 10 at 30°C for 3 days.

CD liquid medium (pH 10)

Glucose: 2%
NaNO$_3$: 0.3%
KCl: 0.2%
KH$_2$PO$_4$: 0.1%
MgSO$_4$·7H$_2$O: 0.05%
FeSO$_4$·7H$_2$O: 0.001%

pH 10

DNAs expressed by culturing under this condition are represented by SEQ ID NOS. shown in the 5th row of Table 1.

(6) High temperature culture condition

The high temperature culture condition means a culture condition wherein a koji mold can grow in a complete medium containing polypeptone or yeast extract, 0.1% to 5% (preferably 2%) glucose, and having a pH of 5 to 7 (preferably pH 6.3) at a culture temperature ranging from 35°C to 42°C (preferably at 37°C), where the koji mold is required to respond or adapt to high temperatures. Under a typical high temperature condition, a koji mold is cultured in the following YPD medium at 37°C for 24 hours.

YPD medium

Yeast extract: 1%
Bacto-pepton: 2%
Adenine sulfate: 0.04%
Glucose: 2%

DNAs expressed by culturing under this condition are represented by SEQ ID NOS. shown in the 6th row of Table 1.

(7) Low temperature culture condition

The low temperature culture condition means a growth condition wherein a koji mold is cultured using a medium, for example, grain comprising solid carbohydrates or protein such as steamed rice, wheat and soybeans, at a culture temperature ranging from 28°C to 33°C (preferably 30°C), for 1 to 3 days (preferably 34 hours), and then the culture temperature is lowered to 20°C to 25°C (preferably 25°C), followed by another 2 to 5 hours (preferably 3 hours) of culturing. Under a typical culture condition, defatted soybeans and roasted and crushed wheat are allowed to absorb water, followed by heat sterilization, and koji mold conidia are inoculated onto the sterilized product and cultured at 30°C for 34 hours for hyphae to grow. Then the temperature is lowered to 25°C, followed by another 3 hours of culturing.
DNAs expressed by culturing under this condition are represented by SEQ ID NOS. shown in the 7th row of Table 1.

(8) Maltose culture condition

The maltose culture condition means a culture condition wherein a koji mold can grow in a complete medium containing polypeptone and the like, 0.1 % to 5% (preferably 2%) maltose, and having a pH of 5 to 7 (preferably pH 6.3) at a culture temperature ranging from 25°C to 33°C (preferably 30°C). Under a typical maltose culture condition, koji mold cells are cultured in the following ACM liquid medium at 30°C for 24 hours, collected by filtration, washed with sterilized water, and then cultured in the following natural medium containing 2% maltose at 30°C for 4 hours.

ACM liquid medium

> Malt extract: 2%
> Bacto-pepton: 0.1 %
> Glucose: 2%

Liquid medium containing 2% maltose

> Bacto-pepton: 1%
> $KH_2PO_4$: 0.5%
> $NANO_3$: 0.1%
> $MgSO_4 \cdot 7H_2O$: 0.05%
> Maltose: 2%

DNAs expressed by culturing under this condition are represented by SEQ ID NOS. shown in the 8th row of Table 1.

(9) Combination of culture conditions

The above culture conditions can be used independently, or can be appropriately combined according to the purpose of detecting *Aspergillus*, the purpose of monitoring various culture conditions, or the like. Examples of such combinations include a combination of the nutrient-rich and the low temperature culture conditions, a combination of the solid and the high temperature culture conditions, a combination of the solid, the high temperature and the maltose culture conditions, a combination of the nutrient-rich, the early germination, the high temperature and the alkaline culture conditions, and all the possible combinations of the above conditions (1) to (8) as long as they are consistent with each other.

4. Designing of probes or primers

Primers for detecting a koji mold, specifically a PCR forward primer (also referred to as a sense primer) and a reverse primer (also referred to as an antisense primer) can be designed and synthesized from the sequences represented by SEQ ID NOS: 1 to 6006. The variety of DNA obtained by culturing under one condition or a combination of the above culture conditions varies depending on each culture condition (see Table 1). A plurality of koji mold DNAs is obtained in each culture condition. Thus, various variety of and a plurality of DNAs selected from such the DNAs are used as the basis for designing the primers of the present invention. For example, a plurality of DNAs is freely selected from koji mold DNAs (Table 1, SEQ ID NOS. shown in the 1st row) obtained by culturing a koji mold under the nutrient-rich culture condition, and then primers are designed from the partial regions of the selected DNAs. In addition, koji mold cells cultured under a combination of a plurality of conditions express DNAs as shown in the rows in Table 1 respectively corresponding to each of the culture conditions combined.

A plurality of DNAs is freely selected from these DNAs, and then primers are designed from the partial regions of the selected DNAs. The minimum and necessary number of primers is determined in consideration of the degree of relationship of closely related species of a koji mold and the degree of mixture. The number of primer sets is at least 1 primer set (2 primers) and preferably 2 to 4 primer sets. Primers can also be designed from characteristic koji mold regions or from regions other than such regions. The primer is 15 to 50 nucleotides in length, preferably 24 to 30 nucleotides in length. The position of DNA for designing the primer is appropriately selected such that 50 to 40,000 bp, and preferably 300 to 1,000 bp fragments are amplified.

The primers of the present invention can be obtained by any general chemical synthesis, for example a chemical synthesis using a DNA autosynthesizer manufactured by Applied Biosystems.

In the meantime, probes for detecting a koji mold can be designed from whole or the partial regions of the above DNAs in a manner similar to that used when the primers for detecting a koji mold are designed. Alternatively, by PCR amplification using the above primers and genomic DNA or cDNA as a template, probes can also be prepared. When a probe is designed from a partial region, the designed probe is 14 to 3000 nucleotides (preferably 20 to 1000 nucleotides) in length.

5. Kit containing primer set and/or probe

The above primer set and/or probe are used as a kit for amplifying or detecting a koji mold-derived DNA. The primer set is used as a kit for amplifying a koji mold-derived DNA together with DNA polymerase (Taq DNA polymerase, Pfu DNA polymerase or the like).

6. PCR, hybridization and detection

A koji mold DNA is amplified using the primer set prepared as described above and DNA polymerase. Template DNA can be prepared by any known method such as a method using cell wall lytic enzyme (e.g., Yatalase, Takara), or a method that involves preparing RNA using guanidium isothiocyanate, and then using a commercially available cDNA synthesis kit (e.g., GibcoBRL).

Amplification is performed by polymerase chain reaction (PCR). Examples of DNA polymerase include AmpliTaq DNA polymerase (Perkin-Elmer), LA Taq DNA polymerase (Takara) and Pfu DNA polymerase (Stratagene).

Each cycle of amplification conditions consists of 90°C to 98°C for 5 seconds to 1 minute (preferably 94°C for 10 seconds to 1 minute) of a denaturation step, 37°C to 68°C for 5 seconds to 3 minutes (preferably 55°C for 30 seconds to 1 minute) of an annealing step, and 65°C to 75°C for 2 seconds to 30 minutes (preferably 72°C for 30 seconds to 1 minute) of an extension step. The amplification cycle is performed 14 to 40 cycles, preferably 30 cycles. To sufficiently denature template DNA and primers, another denaturation step of 94°C for 1 to 3 minutes can be added before the above amplification cycles. To completely extend the amplified DNA, another extension step of 72°C for 2 to 10 minutes may be added after the amplification cycles. Furthermore, when amplification products are not immediately subjected to detection, a step of storing the amplification products at 4°C is preferably added to avoid nonspecific amplification. Moreover, by performing the annealing step and the extension step at the same temperature, reaction time can be simplified and shortened.

Subsequently, the amplification product is subjected to agarose gel electrophoresis, and then stained with ethidium bromide, SYBR Green solution or the like. The amplification product is then detected as a band, so that the presence of each koji mold can be determined. Instead of agarose gel electrophoresis, polyacrylamide gel electrophoresis or capillary electrophoresis may be performed. Moreover, PCR is performed using primers pre-labeled with a fluorescent dye or the like, so that amplification products can also be detected. Furthermore, other detection methods that do not require electrophoresis can be employed herein. Such methods involve causing the amplification products to bind with a solid phase such as a microplate, and then detecting the product using fluorescence, enzyme reaction, or the like.

When hybridization is performed, probes labeled with a radioactive isotope such as $^{32}$P or $^{35}$S or digoxigenin (DIG) are hybridized to sample DNA, and then detection can be performed using autoradiography, an image analyzer or the like.

There are various koji mold mutants (substitution, deletion and insertion) used according to the production purpose, but the details of the mutants have not usually been elucidated. Therefore, with a certain kind of probe, signals to be originally detected cannot often be detected due to the lack of a gene corresponding to the probe. Hence, detection is performed using a large number of probes (to increase redundancy), so as to remove exceptional detection results due to mutation and thus to enable precise detection. The number of probes used in this case is between 1 and 100, and is preferably between 2 and 5.

Therefore, the use of the detection results obtained by the detection method of the present invention as an indicator make it possible to estimate the growth or fermentation states of a koji mold. For example, when obtained detection results show that koji mold spores are at early germination, it can be estimated that the koji mold is in a growth state insufficient for fermentation production. When obtained detection results indicate a nutrient-deficient condition, it can be estimated that the growth state of the koji mold has already passed through the best state. Furthermore, when obtained detection results indicate a nutrient-rich condition, it can be estimated that the koji mold is in a good state for fermentation, and efficient fermentation production can be continued. Moreover, when obtained detection results show that a koji mold is in a solid culture condition, it can be estimated that fermentation of solids is insufficient and there is a need to proceed with fermentation by continuing the fermentation state for the koji mold.

Furthermore, in the case of assay for mutants, mutants wherein only a certain gene expression is abnormal are often found by detection using a large number of probes. Based on the results, a gene directly involved in mutation of the mutant can be identified. This enables the efficient improvement (breeding) of species based on the identified mutation. The number of probes to be used in this case is between 1 and 100, and is preferably between 5 and 20.

BRIEF DESCRIPTION OF THE DRAWINGS

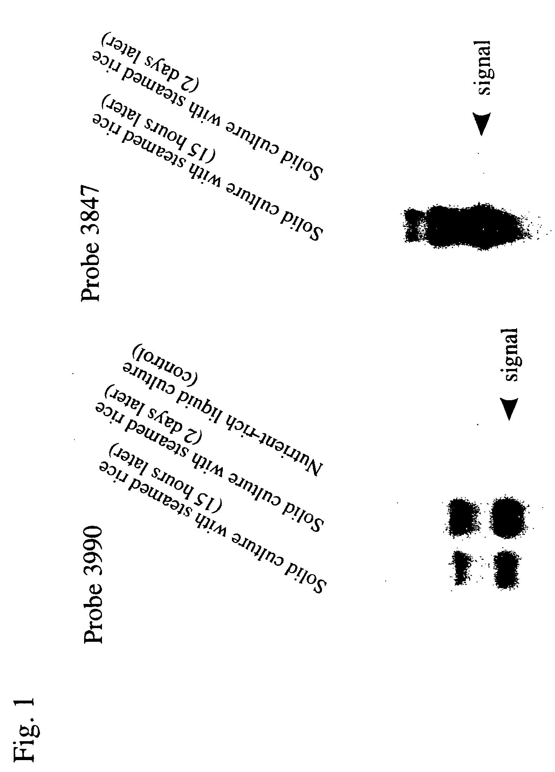

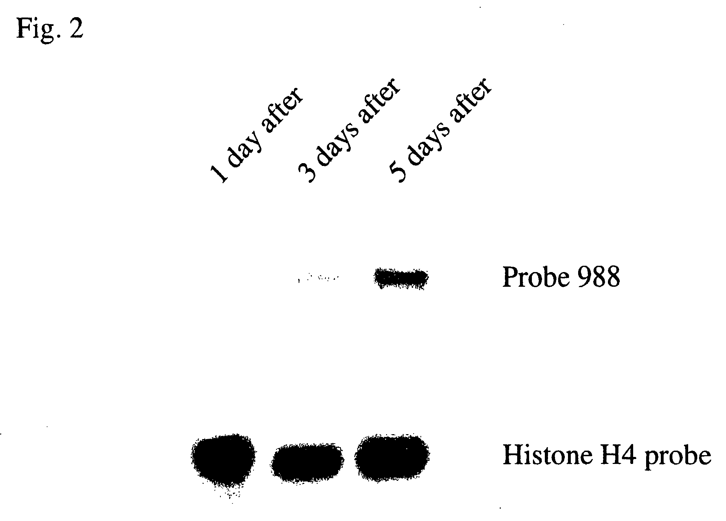

Fig. 1 shows photographs showing the monitored growth states of *Aspergillus* in the fermentation of steamed rice. Fig. 2 shows a photograph showing the monitored nutrient states of *Aspergillus* in the solid fermentation of soybeans.

BEST MODE OF CARRYING OUT THE INVENTION

The present invention will be hereafter described in detail by referring to examples. However, the technical scope of the present invention is not limited by these examples.

[Example 1] Setting for culture condition

(1) Nutrient-rich library

The *Aspergillus oryzae* strain RIB40, a koji mold, was cultured in YPD media (1% yeast extract, 2% bacto-pepton, 0.04% adenine sulfate and 2% glucose) for 22 hours, and then mRNA was prepared from the obtained cells by a method using guanidium thiocyanate. cDNA was synthesized using primers for reverse transcription of a Marathon cDNA synthesis kit (Clontech). The adapter used herein was the *Eco*R I adaptor of the Directional Cloning Tool Box (Pharmacia). The vector used herein was pBluescript SKII+, and the above cDNA was inserted into the *Eco*R I-*Not* I site in the 5' to 3' direction. *Escherichia coli* XLI-Blue was transformed with the vector, plasmids were prepared by the alkaline method, and sequenced using a M13 universal primer.


Primer for reverse transcription

5'-

pTTCTAGAATTCAGCGGCCGCTTTTTTTTTTTTTTTTTTTTTTTTTTTTTTVN-

3' (SEQ ID NO: 6007)


(V = A, C or G, N = A, T, G or C)


*Eco*R I adaptor

5'-AATTCGGCACGAGG-3'

3'-GCCGTGCTCCp-5'


As a result, DNAs having the nucleotide sequences represented by SEQ ID NOS. shown in the 1st row of Table 1 were obtained.

(2) Solid culture library

*Aspergillus* suspended in water for conidia was added to wheat bran, cultured at 30°C for 27 hours, and then the cells were collected. When the state and appearance of the cells at this time were visually observed, aerial hyphae were clearly recognized, but conidia formation had not begun. The cells were disrupted in liquid nitrogen, and then total RNA was separated by the Cathala method. mRNA was separated by the Oligotex method. cDNA was synthesized using a superscript cDNA plasmid system (GIBCO BRL). The vector used herein was pSPORT1 provided with the kit, and the cDNA was inserted into the *Sal* I-*Not* I site. Transformation, plasmid preparation, sequencing methods and the like are the same as those used for the nutrient-rich library.

Primer for reverse transcription

5'-pGACTAGTTCTAGATCGCGAGCGGCCGCCCTTTTTTTTTTTTTTT-3' (SEQ ID NO: 6008)

Sal I adaptor

5'-TCGACCCACGCGTCCG-3'

3'-GGGTGCGCAGGCp-5'

As a result, DNAs having the nucleotide sequences represented by the SEQ ID NOS. shown in the 3rd row of Table 1 were obtained.

(3) High temperature culture library

*Aspergillus* was shake-cultured in YPD media at 37°C for 24 hours, total RNA was extracted from the cells with ISOGEN (NIPPON GENE), and then mRNA was prepared using a mRNA purification kit (Pharmacia). cDNA was synthesized using a cDNA synthesis kit (Pharmacia) and a *Not* I/Oligo-dT$_{18}$ primer of a directional cloning toolbox. The obtained cDNA was incorporated into the *Eco*R I→*Not* I site of pBluescript SKII+. Transformation, plasmid preparation, sequencing methods and the like were the same as those used for the nutrient-rich library.

As a result, DNAs having the nucleotide sequences represented by the SEQ ID NOS. shown in the 6th row of Table 1 were obtained.

(4) Early germination library

Conidia were obtained by culturing *Aspergillus* on PD (potato dextrose agar) plates at 28°C for 8 days. The germinated cells were obtained by culturing conidia in SP liquid media (3.5% soluble starch, 2% polypeptone, 0.5% KH$_2$PO$_4$, 0.5% MgSO$_4$·7H$_2$O) at 30°C for 12 hours. The mixture of conidia and the germinated cells, were disrupted with sea sand in liquid nitrogen, and then mRNA was purified using a QuickPrep Micro mRNA Purification Kit (Amersham Pharmacia Biotech). Subsequently, cDNA was synthesized using a SuperScript™ Choice System for cDNA Synthesis Kit (GIBCO BRL). The vector used herein was pBluescript SKII+, and the cDNA was inserted into the *Eco*R I-*Not* I site. Transformation, plasmid preparation, sequencing methods and the like are the same as those used for the nutrient-rich library.

As a result, DNAs having the nucleotide sequences represented by the SEQ ID NOS. shown in the 4th row of Table 1 were obtained.

(5) Alkaline culture library

Sterilized cellophane was placed on a CD (Czapek-Dox) agar media adjusted to pH 10. Conidia of the *A. oryzae* strain RIB40 were inoculated thereon and cultured at 30°C for 3 days. The cells that had grown on the cellophane were collected, total RNA was prepared using a Total RNA purification kit (QIAGEN), and then mRNA was extracted using a mRNA purification kit (Pharmacia). cDNA was synthesized using a kit (BRL), and then inserted into the *Sal* I-*Not* I site of the vector, pSPORT1. Transformation, plasmid preparation, sequencing methods and the like are the same as those used for the nutrient-rich library.

As a result, DNAs having the nucleotide sequences represented by the SEQ ID NOS. shown in the 5th row of Table 1 were obtained.

(6) Maltose culture library

*Aspergillus* was shake-cultured in ACM media (2% malt extract, 0.1% bacto-pepton and 2% glucose) at 37°C for 24 hours. The cells were collected by filtration, and then washed with natural media (1% bacto-pepton, 0.5% KH$_2$PO$_4$, 0.1% NANO$_3$, 0.05% MgSO$_4$·7H$_2$O). The cells were further shake-cultured in natural media containing 2% maltose at 37°C for 4 hours. RNA was prepared from the thus obtained cells based on the Okayama-Berg method. mRNA was then purified from total RNA using an oligotex dT30 (Super) (Nippon Roche K. K.), and then cDNA was synthesized according to the cDNA synthesis kit (Stratagene). The thus obtained cDNA was incorporated into the pBluescript KS(+) *Eco*R I →*Xho* I site. Transformation, plasmid preparation, sequencing methods and the like are the same as those used for the nutrient-rich library.

As a result, DNAs having the nucleotide sequences represented by the SEQ ID NOS. shown in the 8th row of Table 1 were obtained.

(7) Nutrient-deficient library

Cells were cultured in YPD media (1% yeast extract, 2% bacto-pepton, 0.04% adenine sulfate and 2% glucose) for 22 hours, collected by filtration, and then washed with distilled water. The cells were then transferred to and cultured for 8 hours in CD media (Czapek-Dox) containing no carbon source such as glucose, and then mRNA was prepared from the resulting cells. cDNA was synthesized in a manner similar to that used for the nutrient-rich library. The average length of cDNAs of the library was approximately slightly less than 1.5 kbp.

As a result, DNAs having the nucleotide sequences represented by the SEQ ID NOS. shown in the 2nd row of Table 1 were obtained.

(8) Low temperature culture library

15 ml of water was added to 10 g of defatted soybeans to absorb water, 10 g of roasted and crushed wheat was added to the soybeans, and then the mixture was agitated. The mixture was put into a 500 ml Erlenmeyer flask. The flask was sealed with a biosilico cap, autoclaved for 30 minutes, and then cooled. *Aspergillus oryzae* RIB40 was then inoculated at $1\times10^5$ spores/g. After inoculation, koji making was performed at 30°C (gas phase type) for 34 hours, followed by 3 hours at 25°C. Thus, cDNA was prepared from the RNA of koji at 37 hours after the start of koji making. In addition, at 18 hours and 34 hours after the start of koji making, the Erlenmeyer flask was shaken for agitation. cDNA was synthesized by LD PCR using a SMART cDNA Library Construction Kit (CLONTECH). The vector used herein was λTripEx2 and the cDNA was inserted into the *Sfi* I site. Transformation, plasmid preparation, sequencing methods and the like are the same as those used for the nutrient-rich library.

As a result, DNAs having the nucleotide sequences represented by the SEQ ID NOS. shown in the 7th row of Table 1 were obtained.

[Example 2] Measuring *Aspergillus* growth state

*Aspergillus* conidia were attached to steamed rice for hyphae to grow with water content of 25% at room temperature for 3 days. From the fermentation products at 15 hours and at 2 days after the start of the fermentation production process, RNA was extracted by a method utilizing a combination of a cell wall lytic enzyme and phenol extraction. 1.2% agarose gel electrophoresis containing formaldehyde was then performed. The electrophoresed RNA was transferred onto a nitrocellulose membrane using capillary action. After blocking with a buffer containing casein, the membrane was subjected to hybridization with DIG-labeled RNA probes respectively having an EST sequence, a 3990 (SEQ ID NO: 3990) sequence and a 3847 (SEQ ID NO: 3847) sequence detected under the solid and early germination culture conditions according to the present invention. The membrane was washed several times with a washing solution containing citrate. The membrane was finally washed with 0.2×SSC at 42°C for 15 minutes, and then treated with a buffer containing anti-DIG antibody and alkaline phosphatase complex and allowed to emit light using CDP-Star, a chemoluminescence reagent. This was imaged with a CCD camera provided with a photosensitizer. When the 3990-derived probe was used, no signal was detected under the nutrient-rich liquid culture condition (control experiment). Signals were detected in both cases at hour 15 and day 2 under the condition using steamed rice. In contrast, when the 3847-derived probe was used, strong signals were detected at hour 15, but they had almost completely disappeared by day 2 (Fig. 1).

At 15 hours after the start of fermentation, conidia were actively germinating. At 2 days after the same, germination was completed, hyphae were actively growing, and the fermentation was proceeding in a solid state. According to the present invention, the precise growth state of *Aspergillus* could be determined at the genetic level.

[Example 3] Measurement of *Aspergillus* growth state

*Aspergillus* hyphae were made to adhere to steamed soybeans, and then fermentation was performed with water content of approximately 30% at room temperature. On days 1, 3 and 5 after the start of fermentation, a part of the fermentation mixture was collected, and then RNA was extracted from the filtration residue by a method using a combination of a cell wall lytic enzyme and phenol extraction. The RNA was subjected to formaldehyde-containing agarose gel electrophoresis, and then transferred onto a nitrocellulose membrane. This membrane was blocked according to a standard method, and then hybridized with a DIG-labeled probe containing the EST nucleotide sequence 988 (SEQ ID NO: 988) obtained under the nutrient-deficient culture condition according to the present invention.

As a result, at days 1, 3 and 5 after the start of culturing, signal intensity continuously increased. Particularly on day 5, signal intensity increased greatly (Fig. 2). In contrast, the signal intensity of a histone gene as a control almost remained unchanged. In this emission condition, it is empirically known that a carbon source derived from a carbohydrate decreases due to consumption from days 3 to 4, and along with this decrease, protease is actively produced. Therefore, by the use of the present invention, the expression state of protease that has been empirically found so far can be genetically found.

This specification includes the content as disclosed in the Japanese Patent Application No. 2001-98371, which is a basis for claiming a priority with respect to the present application. All publications, patents and patent applications cited herein are incorporated herein by reference in their entirety.

Industrial Applicability

The present invention provides a method of detecting the expression of a koji mold gene, and DNAs for specifically measuring the fermentation conditions for a koji mold. With conventional methods, measurement has been performed using secondary factors including the color, odor, pH, water content and the like of fermentation products. In contrast, the present invention is useful in that the actual growth states (fermentation states) of a koji mold can be directly and quantitatively measured according to the method of the present invention. Moreover, the present invention extensively includes almost all genes to be expressed in fermentation production by a koji mold, so that the presence of other genes having sequences similar to that of a target gene can be previously predicted. Furthermore, the present invention is also useful in that the expression amount of a target gene can be precisely measured using probes or primers having nucleotide sequences that are absent in genes other than the target, even when other genes having sequences analogous to that of the target gene are expressed in large quantity.

Sequence Listing Free Text

SEQ ID NO: 6007: Synthetic DNA
SEQ ID NO: 6008: Synthetic DNA

**Claims**

1. A DNA (a) or (b) as shown below:

   (a) a DNA comprising a nucleotide sequence represented by any one of SEQ ID NOS: 1 to 6006; and
   (b) a DNA hybridizing under stringent conditions to the DNA comprising the nucleotide sequence represented by any one of SEQ ID NOS: 1 to 6006, which can be expressed in filamentous fungi when the filamentous fungi are cultured under at least one culture condition selected from the group consisting of a nutrient-rich culture, nutrient-deficient culture, solid culture, early germination culture, alkaline culture, high temperature culture, low temperature culture and maltose culture conditions.

2. A DNA that can be expressed in filamentous fungi when filamentous fungi are cultured under at least one culture condition selected from the group consisting of a nutrient-rich culture, nutrient-deficient culture, solid culture, early germination culture, alkaline culture, high temperature culture, low temperature culture and maltose culture conditions.

3. The DNA of claim 2 comprising a nucleotide sequence represented by any one of the SEQ ID NOS. shown in the 1st row of Table 1, which can be expressed in filamentous fungi when the filamentous fungi are cultured under a nutrient-rich culture condition.

4. The DNA of claim 2 comprising a nucleotide sequence represented by any one of the SEQ ID NOS. shown in the 2nd row of Table 1, which can be expressed in filamentous fungi when the filamentous fungi are cultured under a nutrient-deficient culture condition.

5. The DNA of claim 2 comprising a nucleotide sequence represented by any one of the SEQ ID NOS. shown in the 3rd row of Table 1, which can be expressed in filamentous fungi when the filamentous fungi are cultured under a solid culture condition.

**6.** The DNA of claim 2 comprising a nucleotide sequence represented by any one of the SEQ ID NOS. shown in the 4th row of Table 1, which can be expressed in filamentous fungi when the filamentous fungi are cultured under an early germination culture condition.

**7.** The DNA of claim 2 comprising a nucleotide sequence represented by any one of the SEQ ID NOS. shown in the 5th row of Table 1, which can be expressed in filamentous fungi when the filamentous fungi are cultured under an alkaline culture condition.

**8.** The DNA of claim 2 comprising a nucleotide sequence represented by any one of the SEQ ID NOS. shown in the 6th row of Table 1, which can be expressed in filamentous fungi when the filamentous fungi are cultured under a high temperature culture condition.

**9.** The DNA of claim 2 comprising a nucleotide sequence represented by any one of the SEQ ID NOS. shown in the 7th row of Table 1, which can be expressed in filamentous fungi when the filamentous fungi are cultured under a low temperature culture condition.

**10.** The DNA of claim 2 comprising a nucleotide sequence represented by any one of the SEQ ID NOS. shown in the 8th row of Table 1, which can be expressed in filamentous fungi when the filamentous fungi are cultured under a maltose culture condition.

**11.** The DNA of any one of claims 1 to 10, wherein the filamentous fungi are microorganisms belonging to the genus *Aspergillus,* the genus *Penicillium,* the genus *Humicola*, the genus *Trichoderma,* the genus *Mucor* or the genus *Fusarium.*

**12.** The DNA of claim 11, wherein the microorganism belonging to the genus *Aspergillus* is a koji mold.

**13.** The DNA of claim 12, wherein the koji mold is *Aspergillus oryzae.*

**14.** A primer set of at least two primers for amplifying a filamentous fungus gene, wherein the two primers are selected from the group consisting of nucleotide sequences prepared from the whole or the partial regions of a plurality of DNAs selected from the DNAs of claims 1 to 10.

**15.** A probe for detecting a filamentous fungus gene, comprising a nucleotide sequence prepared from the whole or the partial regions of a plurality of DNAs selected from the DNAs of claims 1 to 10.

**16.** A method of detecting filamentous fungi, comprising synthesizing cDNA from RNA extracted from filamentous fungi, amplifying the cDNA using the primer set of claim 14, and detecting a filamentous fungus gene from the obtained amplification product.

**17.** A method of detecting filamentous fungi, comprising hybridizing the probe of claim 15 to RNA extracted from filamentous fungi and detecting a filamentous fungus gene from the obtained product.

**18.** A method of estimating the growth states or the fermentation function of filamentous fungi using the results obtained by the detection method of claim 16 or 17 as an indicator.

**19.** The method of any one of claims 16 to 18, wherein the filamentous fungi are microorganisms belonging to the genus *Aspergillus,* the genus *Penicillium,* the genus *Humicola,* the genus *Trichoderma*, the genus *Mucor* or the genus *Fusarium.*

**20.** The method of claim 19, wherein the microorganism belonging to the genus *Aspergillus* is a koji mold.

**21.** The method of claim 20, wherein the koji mold is *Aspergillus oryzae.*

Fig. 1

Probe 3847

Solid culture with steamed rice
(15 hours later)

Solid culture with steamed rice
(2 days later)

signal

Probe 3990

Solid culture with steamed rice
(15 hours later)

Solid culture with steamed rice
(2 days later)

Nutrient-rich liquid culture
(control)

signal

Fig. 2

# INTERNATIONAL SEARCH REPORT

| International application No. |
|---|
| PCT/IB02/00890 |

**A. CLASSIFICATION OF SUBJECT MATTER**
Int.Cl$^7$ C12N15/31, C12Q1/02, C12Q1/68

According to International Patent Classification (IPC) or to both national classification and IPC

**B. FIELDS SEARCHED**

Minimum documentation searched (classification system followed by classification symbols)
Int.Cl$^7$ C12N15/31, C12Q1/02, C12Q1/68

Documentation searched other than minimum documentation to the extent that such documents are included in the fields searched

Electronic data base consulted during the international search (name of data base and, where practicable, search terms used)
WPI(DIALOG), BIOSIS(DIALOG), MEDLINE(STN), JICST FILE(JOIS), GenBank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
|---|---|---|
| X | Database GenBank Accession No.AA788450, 1998, KUPFER, D. et al. An Aspergillus nidulans EST Database. | 1-3,11-21 |
| X | Masayuki MACHIDA, "Itojokin Genome Kaiseki to Wagakuni ni okeru Kojikin EST Kaiseki", Bioscience & Industry 2000, Vol.58, No.9, pages 639 to 642 | 1-3,11-21 |
| P,X | Masayuki MACHIDA, "Kabi no Bunshi Seibutsugaku 6 Kojikin no EST Kaiseki", Kagaku to Seibutsu 2001, Jun., Vol.39, No.6, pages 384 to 388 | 1-3,11-21 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 10 July, 2002 (10.07.02) | 23 July, 2002 (23.07.02) |

| Name and mailing address of the ISA/ | Authorized officer |
|---|---|
| Japanese Patent Office | |
| Facsimile No. | Telephone No. |

Form PCT/ISA/210 (second sheet) (July 1998)

<center>**INTERNATIONAL SEARCH REPORT**</center>

| International application No. |
|---|
| PCT/IB02/00890 |

---

**Box I    Observations where certain claims were found unsearchable (Continuation of item 1 of first sheet)**

This international search report has not been established in respect of certain claims under Article 17(2)(a) for the following reasons:

1. ☐    Claims Nos.:

    because they relate to subject matter not required to be searched by this Authority, namely:

2. ☐  Claims Nos.:

    because they relate to parts of the international application that do not comply with the prescribed requirements to such an extent that no meaningful international search can be carried out, specifically:

3. ☐  Claims Nos.:

    because they are dependent claims and are not drafted in accordance with the second and third sentences of Rule 6.4(a).

---

**Box II    Observations where unity of invention is lacking (Continuation of item 2 of first sheet)**

This International Searching Authority found multiple inventions in this international application, as follows:

   (See extra sheet.)

1. ☐  As all required additional search fees were timely paid by the applicant, this international search report covers all searchable claims.

2. ☐  As all searchable claims could be searched without effort justifying an additional fee, this Authority did not invite payment of any additional fee.

3. ☐  As only some of the required additional search fees were timely paid by the applicant, this international search report covers only those claims for which fees were paid, specifically claims Nos.:

4. ☒  No required additional search fees were timely paid by the applicant. Consequently, this international search report is restricted to the invention first mentioned in the claims; it is covered by claims Nos.:

   Inventions relating to DNA of SEQ ID No:1 as set forth in claims 1 to 3 and 11 to 21

**Remark on Protest**   ☐   The additional search fees were accompanied by the applicant's protest.

                ☐   No protest accompanied the payment of additional search fees.

---

Form PCT/ISA/210 (continuation of first sheet (1)) (July 1998)

<center>33</center>

**INTERNATIONAL SEARCH REPORT**

International application No.

PCT/IB02/00890

Continuation of Box No.II of continuation of first sheet(1)

The invention as set forth in claim 1 relates to (a) a DNA containing a base sequence represented by any of SEQ ID NOS:1 to 6006, or (b) a DNA which can be expressed by hybridizing with a DNA containing a base sequence represented by any of SEQ ID NOS:1 to 6006 under stringent conditions and culturing a fungus under at least one condition factor selected from the group consisting of eutrophic culture, oligotrophic culture, solid culture, early germination culture, alkaline culture, high-temperature culture, low-temperature culture and maltose culture. Considering the description, it is recognized that DNAs comprising the base sequences represented by SEQ ID NOS:1 to 6006 are DNAs which are expressed when a fungus is cultured under any condition factor selected from among eutrophic culture, oligotrophic culture, solid culture, early germination culture, alkaline culture, high-temperature culture, low-temperature culture and maltose culture. However, DNAs expressed when a fungus is cultured under any condition factor selected from among eutrophic culture, oligotrophic culture, solid culture, early germination culture, alkaline culture, high-temperature culture, low-temperature culture and maltose culture cannot be considered as novel ones, even though they originate in *Aspergillus oryzae* (Bioscience Industry, Vol. 58, No. 9(2000), p. 639-642). Such being the case, the inventions relating to DNAs comprising the base sequences represented by SEQ ID NOS: 1 to 6006 cannot be considered as a group of inventions so linked as to form a single general inventive concept.

.Therefore, the inventions as set forth in claims 1 to 21 are divided into 6006 groups of inventions which cannot be considered as a group of inventions so linked as to form a single general inventive concept.

Form PCT/ISA/210 (extra sheet) (July 1998)